# EUROPEAN PATENT APPLICATION

(11) **EP 3 354 278 A1**
(43) Date of publication of application: **01.08.2018**
(21) Application number: 17305110.3
(22) Date of filing: 31.01.2017
(51) Int. Cl.: A61K 39/00, C07K 16/18, A61K 39/395

(54) **NEURONAL CELL PROTECTIVE EFFECT OF ANTIBODIES SPECIFIC FOR THE PROTOFIBRILLAR FORM OF THE BETA-AMYLOID PEPTIDE**

(71) Applicant: SANOFI, 75008 Paris (FR)
(72) Inventor: TAUPIN, Veronique, 75008 Paris (FR); DEDIEU, Jean-François, 75008 Paris (FR); COHEN, Caroline, 75008 Paris (FR); BERTRAND, Philippe, 75008 Paris (FR)
(74) Representative: Lavoix

(57) **Abstract**

The present invention relates to an antibody specific for the protofibrillar form of the Aβ peptide for use in the prevention and/or the treatment of a neurodegenerative disease involving Aβ peptide aggregation, in particular for the prevention of Alzheimer's disease and/or the treatment of an early stage of symptomatic Alzheimer's disease. The antibody may thus be used before the appearance of memory loss and impaired cognitive functions or at the earliest stages of the disease. Said antibody has indeed a neuronal cell protective effect against Aβ peptide-induced neurotoxicity.

SEQUENCE LISTING

<110>  SANOFI

<120>  NEURONAL CELL PROTECTIVE EFFECT OF ANTIBODIES SPECIFIC FOR THE
       PROTOFIBRILLAR FORM OF THE BETA-AMYLOID PEPTIDE

<130>  BET16P3445

<160>  36

<170>  PatentIn version 3.5

<210>  1
<211>  1326
<212>  DNA
<213>  Artificial Sequence

<220>
<223>  VH1+CH1

<220>
<221>  CDS
<222>  (1)..(1326)

<400>  1
gag gtc cag ctg cag cag tct ggg cct gag gtg gtg aag cct ggg gtc      48
Glu Val Gln Leu Gln Gln Ser Gly Pro Glu Val Val Lys Pro Gly Val
1               5                   10                  15

tca gtg aag att tcc tgc aag ggt tcc ggc tac aca ttc act gat tat      96
Ser Val Lys Ile Ser Cys Lys Gly Ser Gly Tyr Thr Phe Thr Asp Tyr
            20                  25                  30

gct atg cac tgg gtg aag cag agt cct ggc aag agt ctg gag tgg att     144
Ala Met His Trp Val Lys Gln Ser Pro Gly Lys Ser Leu Glu Trp Ile
        35                  40                  45

gga gtt att agt act aag tat ggt aag aca aac tac aac ccc agc ttt     192
Gly Val Ile Ser Thr Lys Tyr Gly Lys Thr Asn Tyr Asn Pro Ser Phe
    50                  55                  60

cag ggc cag gcc aca atg act gtt gac aaa tcc tcc agc aca gcc tat     240
Gln Gly Gln Ala Thr Met Thr Val Asp Lys Ser Ser Ser Thr Ala Tyr
65                  70                  75                  80

atg gag ctt gcc agc ttg aag gcc tcc gat tct gcc atc tat tac tgt     288
Met Glu Leu Ala Ser Leu Lys Ala Ser Asp Ser Ala Ile Tyr Tyr Cys
                85                  90                  95

gca aga ggg gac gat ggt tat tcc tgg ggt caa gga acc tca gtc acc     336
Ala Arg Gly Asp Asp Gly Tyr Ser Trp Gly Gln Gly Thr Ser Val Thr
            100                 105                 110

gtc tcc agc gct tct acc aag ggc cct tcc gtg ttc cct ctg gcc cct     384
Val Ser Ser Ala Ser Thr Lys Gly Pro Ser Val Phe Pro Leu Ala Pro
        115                 120                 125

tgc tcc cgg tcc acc tcc gag tcc acc gcc gct ctg ggc tgc ctg gtg     432
Cys Ser Arg Ser Thr Ser Glu Ser Thr Ala Ala Leu Gly Cys Leu Val
    130                 135                 140

aag gac tac ttc cct gag cct gtg acc gtg tcc tgg aac tct ggc gcc 480
Lys Asp Tyr Phe Pro Glu Pro Val Thr Val Ser Trp Asn Ser Gly Ala
145                 150                 155                 160

ctg acc tcc ggc gtg cac acc ttc cct gcc gtg ctg cag tcc tcc ggc 528
Leu Thr Ser Gly Val His Thr Phe Pro Ala Val Leu Gln Ser Ser Gly
                165                 170                 175

ctg tac tcc ctg tcc tcc gtg gtg acc gtg cct tcc tcc tcc ctg ggc 576
Leu Tyr Ser Leu Ser Ser Val Val Thr Val Pro Ser Ser Ser Leu Gly
            180                 185                 190

acc aag acc tac acc tgt aac gtg gac cac aag cct tcc aac acc aag 624
Thr Lys Thr Tyr Thr Cys Asn Val Asp His Lys Pro Ser Asn Thr Lys
        195                 200                 205

gtg gac aag cgg gtg gag tcc aag tac ggc cct cct tgc cct ccc tgc 672
Val Asp Lys Arg Val Glu Ser Lys Tyr Gly Pro Pro Cys Pro Pro Cys
    210                 215                 220

cct gcc cct gag ttc gag ggc gga cct agc gtg ttc ctg ttc cct cct 720
Pro Ala Pro Glu Phe Glu Gly Gly Pro Ser Val Phe Leu Phe Pro Pro
225                 230                 235                 240

aag cct aag gac acc ctg atg atc tcc cgg acc cct gag gtg acc tgt 768
Lys Pro Lys Asp Thr Leu Met Ile Ser Arg Thr Pro Glu Val Thr Cys
                245                 250                 255

gtg gtg gtg gac gtg tcc cag gag gac cct gag gtc cag ttc aac tgg 816
Val Val Val Asp Val Ser Gln Glu Asp Pro Glu Val Gln Phe Asn Trp
            260                 265                 270

tac gtg gac ggc gtg gag gtg cac aac gcc aag acc aag cct cgg gag 864
Tyr Val Asp Gly Val Glu Val His Asn Ala Lys Thr Lys Pro Arg Glu
        275                 280                 285

gag cag ttc aat tcc acc tac cgg gtg gtg tct gtg ctg acc gtg ctg 912
Glu Gln Phe Asn Ser Thr Tyr Arg Val Val Ser Val Leu Thr Val Leu
    290                 295                 300

cac cag gac tgg ctg aac ggc aaa gaa tac aag tgt aag gtc tcc aac 960
His Gln Asp Trp Leu Asn Gly Lys Glu Tyr Lys Cys Lys Val Ser Asn
305                 310                 315                 320

aag ggc ctg ccc tcc tcc atc gag aaa acc atc tcc aag gcc aag ggc 1008
Lys Gly Leu Pro Ser Ser Ile Glu Lys Thr Ile Ser Lys Ala Lys Gly
                325                 330                 335

cag cct agg gag cct cag gtg tac acc ctg cct cct agc cag gaa gag 1056
Gln Pro Arg Glu Pro Gln Val Tyr Thr Leu Pro Pro Ser Gln Glu Glu
            340                 345                 350

atg acc aag aac cag gtg tcc ctg acc tgt ctg gtg aag ggc ttc tac 1104
Met Thr Lys Asn Gln Val Ser Leu Thr Cys Leu Val Lys Gly Phe Tyr
        355                 360                 365

cct tcc gac atc gcc gtg gag tgg gag tcc aac ggc cag cct gag aac 1152
Pro Ser Asp Ile Ala Val Glu Trp Glu Ser Asn Gly Gln Pro Glu Asn
    370                 375                 380

aac tac aag acc acc cct cct gtg ctg gac tcc gac ggc tcc ttc ttc 1200
Asn Tyr Lys Thr Thr Pro Pro Val Leu Asp Ser Asp Gly Ser Phe Phe
385                 390                 395                 400

ctg tac tcc agg ctg acc gtg gac aag tcc cgg tgg cag gag ggc aac 1248
Leu Tyr Ser Arg Leu Thr Val Asp Lys Ser Arg Trp Gln Glu Gly Asn
                405                 410                 415

gtc ttt tcc tgc tcc gtg atg cac gag gcc ctg cac aac cac tac acc 1296
Val Phe Ser Cys Ser Val Met His Glu Ala Leu His Asn His Tyr Thr
            420                 425                 430

cag aag tcc ctg tcc ctg tct ctg ggc tga 1326
Gln Lys Ser Leu Ser Leu Ser Leu Gly
        435                 440

<210> 2
<211> 441
<212> PRT
<213> Artificial Sequence

<220>
<223> Synthetic Construct

<400> 2

Glu Val Gln Leu Gln Gln Ser Gly Pro Glu Val Val Lys Pro Gly Val
1               5                   10                  15

Ser Val Lys Ile Ser Cys Lys Gly Ser Gly Tyr Thr Phe Thr Asp Tyr
            20                  25                  30

Ala Met His Trp Val Lys Gln Ser Pro Gly Lys Ser Leu Glu Trp Ile
        35                  40                  45

Gly Val Ile Ser Thr Lys Tyr Gly Lys Thr Asn Tyr Asn Pro Ser Phe
    50                  55                  60

Gln Gly Gln Ala Thr Met Thr Val Asp Lys Ser Ser Ser Thr Ala Tyr
65                  70                  75                  80

Met Glu Leu Ala Ser Leu Lys Ala Ser Asp Ser Ala Ile Tyr Tyr Cys
                85                  90                  95

Ala Arg Gly Asp Asp Gly Tyr Ser Trp Gly Gln Gly Thr Ser Val Thr
            100                 105                 110

Val Ser Ser Ala Ser Thr Lys Gly Pro Ser Val Phe Pro Leu Ala Pro
        115                 120                 125

Cys Ser Arg Ser Thr Ser Glu Ser Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Lys Thr Tyr Thr Cys Asn Val Asp His Lys Pro Ser Asn Thr Lys
        195                 200                 205

Val Asp Lys Arg Val Glu Ser Lys Tyr Gly Pro Pro Cys Pro Pro Cys
    210                 215                 220

Pro Ala Pro Glu Phe Glu Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser Gln Glu Asp Pro Glu Val Gln Phe Asn Trp
            260                 265                 270

Tyr Val Asp Gly Val Glu Val His Asn Ala Lys Thr Lys Pro Arg Glu
        275                 280                 285

Glu Gln Phe Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Gly Leu Pro Ser Ser Ile Glu Lys Thr Ile Ser Lys Ala Lys Gly
                325                 330                 335

Gln Pro Arg Glu Pro Gln Val Tyr Thr Leu Pro Pro Ser Gln Glu Glu
            340                 345                 350

Met Thr Lys Asn Gln Val Ser Leu Thr Cys Leu Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Tyr Ser Arg Leu Thr Val Asp Lys Ser Arg Trp Gln Glu Gly Asn
                405                 410                 415

Val Phe Ser Cys Ser Val Met His Glu Ala Leu His Asn His Tyr Thr
            420                 425                 430

Gln Lys Ser Leu Ser Leu Ser Leu Gly
        435                 440

<210>  3
<211>  660
<212>  DNA
<213>  Artificial Sequence

<220>
<223>  VL1+CL1

<220>
<221>  CDS
<222>  (1)..(660)

<400>  3
gag atc gtg atg acc caa act cca ctc tcc ctg cct gtc agt ctt gga      48
Glu Ile Val Met Thr Gln Thr Pro Leu Ser Leu Pro Val Ser Leu Gly
1               5                   10                  15

gat aga gcc tcc atc tct tgc aga tct ggt cag agc ctt gtg cac agt      96
Asp Arg Ala Ser Ile Ser Cys Arg Ser Gly Gln Ser Leu Val His Ser
            20                  25                  30

aat gga aac acc tat ctg cat tgg tac ctg cag aag cca ggc cag tct      144
Asn Gly Asn Thr Tyr Leu His Trp Tyr Leu Gln Lys Pro Gly Gln Ser
        35                  40                  45

cca aag ctc ctg atc tat aca gtt tcc aac cga ttt tct ggg gtc ccg      192
Pro Lys Leu Leu Ile Tyr Thr Val Ser Asn Arg Phe Ser Gly Val Pro
    50                  55                  60

gac agg ttc agt ggc agt gga tca ggg tca gat ttc aca ctc acc atc      240
Asp Arg Phe Ser Gly Ser Gly Ser Gly Ser Asp Phe Thr Leu Thr Ile
65                  70                  75                  80

agc aga gtg gag gct gag gat ctg gga gtt tat ttc tgc tct caa aat      288
Ser Arg Val Glu Ala Glu Asp Leu Gly Val Tyr Phe Cys Ser Gln Asn
                85                  90                  95

aca ttt gtt cct tgg acg ttc ggt gga ggc acc aag ctg gaa atc aaa      336
Thr Phe Val Pro Trp Thr Phe Gly Gly Gly Thr Lys Leu Glu Ile Lys
            100                 105                 110

cgt acg gtg gct gca cca tct gtc ttc atc ttc ccg cca tct gat gag      384
Arg Thr Val Ala Ala Pro Ser Val Phe Ile Phe Pro Pro Ser Asp Glu
        115                 120                 125

cag ttg aaa tct gga act gcc tct gtt gtg tgc ctg ctg aat aac ttc      432
Gln Leu Lys Ser Gly Thr Ala Ser Val Val Cys Leu Leu Asn Asn Phe
    130                 135                 140

tat ccc aga gag gcc aaa gta cag tgg aag gtg gat aac gcc ctc caa      480
Tyr Pro Arg Glu Ala Lys Val Gln Trp Lys Val Asp Asn Ala Leu Gln
145                 150                 155                 160

tcg ggt aac tcc cag gag agt gtc aca gag cag gac agc aag gac agc 528
Ser Gly Asn Ser Gln Glu Ser Val Thr Glu Gln Asp Ser Lys Asp Ser
                165                 170                 175

acc tac agc ctc agc agc acc ctg acg ctg agc aaa gca gac tac gag 576
Thr Tyr Ser Leu Ser Ser Thr Leu Thr Leu Ser Lys Ala Asp Tyr Glu
            180                 185                 190

aaa cac aaa gtc tac gcc tgc gaa gtc acc cat cag ggc ctg agc tcg 624
Lys His Lys Val Tyr Ala Cys Glu Val Thr His Gln Gly Leu Ser Ser
        195                 200                 205

ccc gtc aca aag agc ttc aac agg gga gag tgt tga 660
Pro Val Thr Lys Ser Phe Asn Arg Gly Glu Cys
    210                 215

<210> 4
<211> 219
<212> PRT
<213> Artificial Sequence

<220>
<223> Synthetic Construct

<400> 4

Glu Ile Val Met Thr Gln Thr Pro Leu Ser Leu Pro Val Ser Leu Gly
1               5                   10                  15

Asp Arg Ala Ser Ile Ser Cys Arg Ser Gly Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Thr Val Ser Asn Arg Phe Ser Gly Val Pro
    50                  55                  60

Asp Arg Phe Ser Gly Ser Gly Ser Gly Ser Asp Phe Thr Leu Thr Ile
65                  70                  75                  80

Ser Arg Val Glu Ala Glu Asp Leu Gly Val Tyr Phe Cys Ser Gln Asn
                85                  90                  95

Thr Phe Val Pro Trp Thr Phe Gly Gly Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  5
<211>  345
<212>  DNA
<213>  Artificial Sequence

<220>
<223>  VH1

<220>
<221>  CDS
<222>  (1)..(345)

<400>  5
gag gtc cag ctg cag cag tct ggg cct gag gtg gtg aag cct ggg gtc       48
Glu Val Gln Leu Gln Gln Ser Gly Pro Glu Val Val Lys Pro Gly Val
1               5                   10                  15

tca gtg aag att tcc tgc aag ggt tcc ggc tac aca ttc act gat tat       96
Ser Val Lys Ile Ser Cys Lys Gly Ser Gly Tyr Thr Phe Thr Asp Tyr
            20                  25                  30

gct atg cac tgg gtg aag cag agt cct ggc aag agt ctg gag tgg att      144
Ala Met His Trp Val Lys Gln Ser Pro Gly Lys Ser Leu Glu Trp Ile
        35                  40                  45

gga gtt att agt act aag tat ggt aag aca aac tac aac ccc agc ttt      192
Gly Val Ile Ser Thr Lys Tyr Gly Lys Thr Asn Tyr Asn Pro Ser Phe
    50                  55                  60

cag ggc cag gcc aca atg act gtt gac aaa tcc tcc agc aca gcc tat      240
Gln Gly Gln Ala Thr Met Thr Val Asp Lys Ser Ser Ser Thr Ala Tyr
65                  70                  75                  80

atg gag ctt gcc agc ttg aag gcc tcc gat tct gcc atc tat tac tgt      288
Met Glu Leu Ala Ser Leu Lys Ala Ser Asp Ser Ala Ile Tyr Tyr Cys
                85                  90                  95

gca aga ggg gac gat ggt tat tcc tgg ggt caa gga acc tca gtc acc      336
Ala Arg Gly Asp Asp Gly Tyr Ser Trp Gly Gln Gly Thr Ser Val Thr
            100                 105                 110

gtc tcc agc                                                          345
Val Ser Ser
        115

<210>  6
<211>  115
<212>  PRT
<213>  Artificial Sequence

<220>
<223>  Synthetic Construct

<400>  6

Glu Val Gln Leu Gln Gln Ser Gly Pro Glu Val Val Lys Pro Gly Val
1               5                   10                  15

Ser Val Lys Ile Ser Cys Lys Gly Ser Gly Tyr Thr Phe Thr Asp Tyr
            20                  25                  30

Ala Met His Trp Val Lys Gln Ser Pro Gly Lys Ser Leu Glu Trp Ile
        35                  40                  45

Gly Val Ile Ser Thr Lys Tyr Gly Lys Thr Asn Tyr Asn Pro Ser Phe
    50                  55                  60

Gln Gly Gln Ala Thr Met Thr Val Asp Lys Ser Ser Ser Thr Ala Tyr
65                  70                  75                  80

Met Glu Leu Ala Ser Leu Lys Ala Ser Asp Ser Ala Ile Tyr Tyr Cys
                85                  90                  95

Ala Arg Gly Asp Asp Gly Tyr Ser Trp Gly Gln Gly Thr Ser Val Thr
            100                 105                 110

Val Ser Ser
        115

<210>  7
<211>  339
<212>  DNA
<213>  Artificial Sequence

<220>
<223>  VL1

<220>
<221>  CDS
<222>  (1)..(339)

<400>  7
gag atc gtg atg acc caa act cca ctc tcc ctg cct gtc agt ctt gga      48
Glu Ile Val Met Thr Gln Thr Pro Leu Ser Leu Pro Val Ser Leu Gly

1               5                   10                  15

gat aga gcc tcc atc tct tgc aga tct ggt cag agc ctt gtg cac agt          96
Asp Arg Ala Ser Ile Ser Cys Arg Ser Gly Gln Ser Leu Val His Ser
            20                  25                  30

aat gga aac acc tat ctg cat tgg tac ctg cag aag cca ggc cag tct          144
Asn Gly Asn Thr Tyr Leu His Trp Tyr Leu Gln Lys Pro Gly Gln Ser
        35                  40                  45

cca aag ctc ctg atc tat aca gtt tcc aac cga ttt tct ggg gtc ccg          192
Pro Lys Leu Leu Ile Tyr Thr Val Ser Asn Arg Phe Ser Gly Val Pro
    50                  55                  60

gac agg ttc agt ggc agt gga tca ggg tca gat ttc aca ctc acc atc          240
Asp Arg Phe Ser Gly Ser Gly Ser Gly Ser Asp Phe Thr Leu Thr Ile
65                  70                  75                  80

agc aga gtg gag gct gag gat ctg gga gtt tat ttc tgc tct caa aat          288
Ser Arg Val Glu Ala Glu Asp Leu Gly Val Tyr Phe Cys Ser Gln Asn
                85                  90                  95

aca ttt gtt cct tgg acg ttc ggt gga ggc acc aag ctg gaa atc aaa          336
Thr Phe Val Pro Trp Thr Phe Gly Gly Gly Thr Lys Leu Glu Ile Lys
            100                 105                 110

cgt                                                                      339
Arg

<210>  8
<211>  113
<212>  PRT
<213>  Artificial Sequence

<220>
<223>  Synthetic Construct

<400>  8

Glu Ile Val Met Thr Gln Thr Pro Leu Ser Leu Pro Val Ser Leu Gly
1               5                   10                  15

Asp Arg Ala Ser Ile Ser Cys Arg Ser Gly Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Thr Val Ser Asn Arg Phe Ser Gly Val Pro
    50                  55                  60

Asp Arg Phe Ser Gly Ser Gly Ser Gly Ser Asp Phe Thr Leu Thr Ile
65                  70                  75                  80

Ser Arg Val Glu Ala Glu Asp Leu Gly Val Tyr Phe Cys Ser Gln Asn
                85                  90                  95

Thr Phe Val Pro Trp Thr Phe Gly Gly Gly Thr Lys Leu Glu Ile Lys
            100                 105                 110

Arg

<210>  9
<211>  33
<212>  DNA
<213>  Artificial Sequence

<220>
<223>  CDR1 VH1

<220>
<221>  CDS
<222>  (1)..(33)

<400>  9
tcc ggc tac aca ttc act gat tat gct atg cac                          33
Ser Gly Tyr Thr Phe Thr Asp Tyr Ala Met His
1               5                   10

<210>  10
<211>  11
<212>  PRT
<213>  Artificial Sequence

<220>
<223>  Synthetic Construct

<400>  10

Ser Gly Tyr Thr Phe Thr Asp Tyr Ala Met His
1               5                   10

<210>  11
<211>  30
<212>  DNA
<213>  Artificial Sequence

<220>
<223>  CDR2 VH1

<220>
<221>  CDS
<222>  (1)..(30)

<400>  11
gtt att agt act aag tat ggt aag aca aac                              30
Val Ile Ser Thr Lys Tyr Gly Lys Thr Asn
1               5                   10

<210>  12

<211>  10
<212>  PRT
<213>  Artificial Sequence

<220>
<223>  Synthetic Construct

<400>  12

Val Ile Ser Thr Lys Tyr Gly Lys Thr Asn
1               5                   10


<210>  13
<211>  18
<212>  DNA
<213>  Artificial Sequence

<220>
<223>  CDR3 VH1


<220>
<221>  CDS
<222>  (1)..(18)

<400>  13
ggg gac gat ggt tat tcc                                                18
Gly Asp Asp Gly Tyr Ser
1               5


<210>  14
<211>  6
<212>  PRT
<213>  Artificial Sequence

<220>
<223>  Synthetic Construct

<400>  14

Gly Asp Asp Gly Tyr Ser
1               5


<210>  15
<211>  48
<212>  DNA
<213>  Artificial Sequence

<220>
<223>  CDR1 VL1


<220>
<221>  CDS
<222>  (1)..(48)

<400>  15
aga tct ggt cag agc ctt gtg cac agt aat gga aac acc tat ctg cat        48
Arg Ser Gly Gln Ser Leu Val His Ser Asn Gly Asn Thr Tyr Leu His
1               5                   10                  15

<210>  16
<211>  16
<212>  PRT
<213>  Artificial Sequence

<220>
<223>  Synthetic Construct

<400>  16

Arg Ser Gly Gln Ser Leu Val His Ser Asn Gly Asn Thr Tyr Leu His
1               5                   10                  15


<210>  17
<211>  24
<212>  DNA
<213>  Artificial Sequence

<220>
<223>  CDR2 VL1


<220>
<221>  CDS
<222>  (1)..(24)

<400>  17
aca gtt tcc aac cga ttt tct ggg                                          24
Thr Val Ser Asn Arg Phe Ser Gly
1               5


<210>  18
<211>  8
<212>  PRT
<213>  Artificial Sequence

<220>
<223>  Synthetic Construct

<400>  18

Thr Val Ser Asn Arg Phe Ser Gly
1               5


<210>  19
<211>  27
<212>  DNA
<213>  Artificial Sequence

<220>
<223>  CDR3 VL1


<220>
<221>  CDS
<222>  (1)..(27)

<400>  19

tct caa aat aca ttt gtt cct tgg acg                                    27
Ser Gln Asn Thr Phe Val Pro Trp Thr
1               5

<210>  20
<211>  9
<212>  PRT
<213>  Artificial Sequence

<220>
<223>  Synthetic Construct

<400>  20

Ser Gln Asn Thr Phe Val Pro Trp Thr
1               5

<210>  21
<211>  660
<212>  DNA
<213>  Artificial Sequence

<220>
<223>  VL2 + CL2

<220>
<221>  CDS
<222>  (1)..(660)

<400>  21
gag atc gtg atg acc caa act cca ctc tcc ctg cct gtc agt ctt gga        48
Glu Ile Val Met Thr Gln Thr Pro Leu Ser Leu Pro Val Ser Leu Gly
1               5                   10                  15

gat aga gcc tcc atc tct tgc aga tct ggt cag agc ctt gtg cac agt        96
Asp Arg Ala Ser Ile Ser Cys Arg Ser Gly Gln Ser Leu Val His Ser
            20                  25                  30

aat acc aac acc tat ctg cat tgg tac ctg cag aag cca ggc cag tct       144
Asn Thr Asn Thr Tyr Leu His Trp Tyr Leu Gln Lys Pro Gly Gln Ser
        35                  40                  45

cca aag ctc ctg atc tat aca gtt tcc aac cga ttt tct ggg gtc ccg       192
Pro Lys Leu Leu Ile Tyr Thr Val Ser Asn Arg Phe Ser Gly Val Pro
    50                  55                  60

gac agg ttc agt ggc agt gga tca ggg tca gat ttc aca ctc acc atc       240
Asp Arg Phe Ser Gly Ser Gly Ser Gly Ser Asp Phe Thr Leu Thr Ile
65                  70                  75                  80

agc aga gtg gag gct gag gat ctg gga gtt tat ttc tgc tct caa aat       288
Ser Arg Val Glu Ala Glu Asp Leu Gly Val Tyr Phe Cys Ser Gln Asn
                85                  90                  95

aca ttt gtt cct tgg acg ttc ggt gga ggc acc aag ctg gaa atc aaa       336
Thr Phe Val Pro Trp Thr Phe Gly Gly Gly Thr Lys Leu Glu Ile Lys
            100                 105                 110

cgt acg gtg gct gca cca tct gtc ttc atc ttc ccg cca tct gat gag       384
Arg Thr Val Ala Ala Pro Ser Val Phe Ile Phe Pro Pro Ser Asp Glu

115                 120                 125

cag ttg aaa tct gga act gcc tct gtt gtg tgc ctg ctg aat aac ttc        432
Gln Leu Lys Ser Gly Thr Ala Ser Val Val Cys Leu Leu Asn Asn Phe
    130                 135                 140

tat ccc aga gag gcc aaa gta cag tgg aag gtg gat aac gcc ctc caa        480
Tyr Pro Arg Glu Ala Lys Val Gln Trp Lys Val Asp Asn Ala Leu Gln
145                 150                 155                 160

tcg ggt aac tcc cag gag agt gtc aca gag cag gac agc aag gac agc        528
Ser Gly Asn Ser Gln Glu Ser Val Thr Glu Gln Asp Ser Lys Asp Ser
                165                 170                 175

acc tac agc ctc agc agc acc ctg acg ctg agc aaa gca gac tac gag        576
Thr Tyr Ser Leu Ser Ser Thr Leu Thr Leu Ser Lys Ala Asp Tyr Glu
            180                 185                 190

aaa cac aaa gtc tac gcc tgc gaa gtc acc cat cag ggc ctg agc tcg        624
Lys His Lys Val Tyr Ala Cys Glu Val Thr His Gln Gly Leu Ser Ser
        195                 200                 205

ccc gtc aca aag agc ttc aac agg gga gag tgt tga                        660
Pro Val Thr Lys Ser Phe Asn Arg Gly Glu Cys
    210                 215

<210>  22
<211>  219
<212>  PRT
<213>  Artificial Sequence

<220>
<223>  Synthetic Construct

<400>  22

Glu Ile Val Met Thr Gln Thr Pro Leu Ser Leu Pro Val Ser Leu Gly
1               5                   10                  15

Asp Arg Ala Ser Ile Ser Cys Arg Ser Gly Gln Ser Leu Val His Ser
            20                  25                  30

Asn Thr Asn Thr Tyr Leu His Trp Tyr Leu Gln Lys Pro Gly Gln Ser
        35                  40                  45

Pro Lys Leu Leu Ile Tyr Thr Val Ser Asn Arg Phe Ser Gly Val Pro
    50                  55                  60

Asp Arg Phe Ser Gly Ser Gly Ser Gly Ser Asp Phe Thr Leu Thr Ile
65                  70                  75                  80

Ser Arg Val Glu Ala Glu Asp Leu Gly Val Tyr Phe Cys Ser Gln Asn
                85                  90                  95

Thr Phe Val Pro Trp Thr Phe Gly Gly Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  23
<211>  339
<212>  DNA
<213>  Artificial Sequence

<220>
<223>  VL2

<220>
<221>  CDS
<222>  (1)..(339)

<400>  23
gag atc gtg atg acc caa act cca ctc tcc ctg cct gtc agt ctt gga        48
Glu Ile Val Met Thr Gln Thr Pro Leu Ser Leu Pro Val Ser Leu Gly
1               5                   10                  15

gat aga gcc tcc atc tct tgc aga tct ggt cag agc ctt gtg cac agt        96
Asp Arg Ala Ser Ile Ser Cys Arg Ser Gly Gln Ser Leu Val His Ser
            20                  25                  30

aat acc aac acc tat ctg cat tgg tac ctg cag aag cca ggc cag tct       144
Asn Thr Asn Thr Tyr Leu His Trp Tyr Leu Gln Lys Pro Gly Gln Ser
        35                  40                  45

cca aag ctc ctg atc tat aca gtt tcc aac cga ttt tct ggg gtc ccg       192
Pro Lys Leu Leu Ile Tyr Thr Val Ser Asn Arg Phe Ser Gly Val Pro
    50                  55                  60

gac agg ttc agt ggc agt gga tca ggg tca gat ttc aca ctc acc atc       240
Asp Arg Phe Ser Gly Ser Gly Ser Gly Ser Asp Phe Thr Leu Thr Ile
65                  70                  75                  80

agc aga gtg gag gct gag gat ctg gga gtt tat ttc tgc tct caa aat     288
Ser Arg Val Glu Ala Glu Asp Leu Gly Val Tyr Phe Cys Ser Gln Asn
                85                  90                  95

aca ttt gtt cct tgg acg ttc ggt gga ggc acc aag ctg gaa atc aaa     336
Thr Phe Val Pro Trp Thr Phe Gly Gly Gly Thr Lys Leu Glu Ile Lys
            100                 105                 110

cgt                                                                 339
Arg

<210>  24
<211>  113
<212>  PRT
<213>  Artificial Sequence

<220>
<223>  Synthetic Construct

<400>  24

Glu Ile Val Met Thr Gln Thr Pro Leu Ser Leu Pro Val Ser Leu Gly
1               5                   10                  15

Asp Arg Ala Ser Ile Ser Cys Arg Ser Gly Gln Ser Leu Val His Ser
            20                  25                  30

Asn Thr Asn Thr Tyr Leu His Trp Tyr Leu Gln Lys Pro Gly Gln Ser
        35                  40                  45

Pro Lys Leu Leu Ile Tyr Thr Val Ser Asn Arg Phe Ser Gly Val Pro
    50                  55                  60

Asp Arg Phe Ser Gly Ser Gly Ser Gly Ser Asp Phe Thr Leu Thr Ile
65                  70                  75                  80

Ser Arg Val Glu Ala Glu Asp Leu Gly Val Tyr Phe Cys Ser Gln Asn
                85                  90                  95

Thr Phe Val Pro Trp Thr Phe Gly Gly Gly Thr Lys Leu Glu Ile Lys
            100                 105                 110

Arg

<210>  25
<211>  1326
<212>  DNA
<213>  Artificial Sequence

<220>
<223>  VH2+ CH2

<220>
<221>  CDS
<222>  (1)..(1326)

<400>  25
gag gtc cag ctg cag cag tct ggg cct gag gtg gtg aag cct ggg gtc      48
Glu Val Gln Leu Gln Gln Ser Gly Pro Glu Val Val Lys Pro Gly Val
1               5                   10                  15

tca gtg aag att tcc tgc aag ggt tcc ggc tac aca ttc act gat tat      96
Ser Val Lys Ile Ser Cys Lys Gly Ser Gly Tyr Thr Phe Thr Asp Tyr
            20                  25                  30

gct atg cac tgg gtg aag cag agt cct ggc aag agt ctg gag tgg att      144
Ala Met His Trp Val Lys Gln Ser Pro Gly Lys Ser Leu Glu Trp Ile
        35                  40                  45

gga gtt att agt act aag tat ggt aag aca aac tac aac ccc agc ttt      192
Gly Val Ile Ser Thr Lys Tyr Gly Lys Thr Asn Tyr Asn Pro Ser Phe
    50                  55                  60

cag ggc cag gcc aca atg act gtt gac aaa tcc tcc agc aca gcc tat      240
Gln Gly Gln Ala Thr Met Thr Val Asp Lys Ser Ser Ser Thr Ala Tyr
65                  70                  75                  80

atg gag ctt gcc agc ttg aag gcc tcc gat tct gcc atc tat tac tgt      288
Met Glu Leu Ala Ser Leu Lys Ala Ser Asp Ser Ala Ile Tyr Tyr Cys
                85                  90                  95

gca aga ggg gac gag ggt tat tcc tgg ggt caa gga acc tca gtc acc      336
Ala Arg Gly Asp Glu Gly Tyr Ser Trp Gly Gln Gly Thr Ser Val Thr
            100                 105                 110

gtc tcc agc gct tct acc aag ggc cct tcc gtg ttc cct ctg gcc cct      384
Val Ser Ser Ala Ser Thr Lys Gly Pro Ser Val Phe Pro Leu Ala Pro
        115                 120                 125

tgc tcc cgg tcc acc tcc gag tcc acc gcc gct ctg ggc tgc ctg gtg      432
Cys Ser Arg Ser Thr Ser Glu Ser Thr Ala Ala Leu Gly Cys Leu Val
    130                 135                 140

aag gac tac ttc cct gag cct gtg acc gtg tcc tgg aac tct ggc gcc      480
Lys Asp Tyr Phe Pro Glu Pro Val Thr Val Ser Trp Asn Ser Gly Ala
145                 150                 155                 160

ctg acc tcc ggc gtg cac acc ttc cct gcc gtg ctg cag tcc tcc ggc      528
Leu Thr Ser Gly Val His Thr Phe Pro Ala Val Leu Gln Ser Ser Gly
                165                 170                 175

ctg tac tcc ctg tcc tcc gtg gtg acc gtg cct tcc tcc tcc ctg ggc      576
Leu Tyr Ser Leu Ser Ser Val Val Thr Val Pro Ser Ser Ser Leu Gly
            180                 185                 190

acc aag acc tac acc tgt aac gtg gac cac aag cct tcc aac acc aag      624
Thr Lys Thr Tyr Thr Cys Asn Val Asp His Lys Pro Ser Asn Thr Lys
        195                 200                 205

gtg gac aag cgg gtg gag tcc aag tac ggc cct cct tgc cct ccc tgc      672
Val Asp Lys Arg Val Glu Ser Lys Tyr Gly Pro Pro Cys Pro Pro Cys
    210                 215                 220

cct gcc cct gag ttc gag ggc gga cct agc gtg ttc ctg ttc cct cct          720
Pro Ala Pro Glu Phe Glu Gly Gly Pro Ser Val Phe Leu Phe Pro Pro
225                 230                 235                 240

aag cct aag gac acc ctg atg atc tcc cgg acc cct gag gtg acc tgt          768
Lys Pro Lys Asp Thr Leu Met Ile Ser Arg Thr Pro Glu Val Thr Cys
                245                 250                 255

gtg gtg gtg gac gtg tcc cag gag gac cct gag gtc cag ttc aac tgg          816
Val Val Val Asp Val Ser Gln Glu Asp Pro Glu Val Gln Phe Asn Trp
            260                 265                 270

tac gtg gac ggc gtg gag gtg cac aac gcc aag acc aag cct cgg gag          864
Tyr Val Asp Gly Val Glu Val His Asn Ala Lys Thr Lys Pro Arg Glu
        275                 280                 285

gag cag ttc aat tcc acc tac cgg gtg gtg tct gtg ctg acc gtg ctg          912
Glu Gln Phe Asn Ser Thr Tyr Arg Val Val Ser Val Leu Thr Val Leu
    290                 295                 300

cac cag gac tgg ctg aac ggc aaa gaa tac aag tgt aag gtc tcc aac          960
His Gln Asp Trp Leu Asn Gly Lys Glu Tyr Lys Cys Lys Val Ser Asn
305                 310                 315                 320

aag ggc ctg ccc tcc tcc atc gag aaa acc atc tcc aag gcc aag ggc          1008
Lys Gly Leu Pro Ser Ser Ile Glu Lys Thr Ile Ser Lys Ala Lys Gly
                325                 330                 335

cag cct agg gag cct cag gtg tac acc ctg cct cct agc cag gaa gag          1056
Gln Pro Arg Glu Pro Gln Val Tyr Thr Leu Pro Pro Ser Gln Glu Glu
            340                 345                 350

atg acc aag aac cag gtg tcc ctg acc tgt ctg gtg aag ggc ttc tac          1104
Met Thr Lys Asn Gln Val Ser Leu Thr Cys Leu Val Lys Gly Phe Tyr
        355                 360                 365

cct tcc gac atc gcc gtg gag tgg gag tcc aac ggc cag cct gag aac          1152
Pro Ser Asp Ile Ala Val Glu Trp Glu Ser Asn Gly Gln Pro Glu Asn
    370                 375                 380

aac tac aag acc acc cct cct gtg ctg gac tcc gac ggc tcc ttc ttc          1200
Asn Tyr Lys Thr Thr Pro Pro Val Leu Asp Ser Asp Gly Ser Phe Phe
385                 390                 395                 400

ctg tac tcc agg ctg acc gtg gac aag tcc cgg tgg cag gag ggc aac          1248
Leu Tyr Ser Arg Leu Thr Val Asp Lys Ser Arg Trp Gln Glu Gly Asn
                405                 410                 415

gtc ttt tcc tgc tcc gtg atg cac gag gcc ctg cac aac cac tac acc          1296
Val Phe Ser Cys Ser Val Met His Glu Ala Leu His Asn His Tyr Thr
            420                 425                 430

cag aag tcc ctg tcc ctg tct ctg ggc tga                                  1326
Gln Lys Ser Leu Ser Leu Ser Leu Gly
        435                 440


<210>  26
<211>  441
<212>  PRT
<213>  Artificial Sequence

<220>

<223>  Synthetic Construct

<400>  26

Glu Val Gln Leu Gln Gln Ser Gly Pro Glu Val Val Lys Pro Gly Val
1               5                   10                  15

Ser Val Lys Ile Ser Cys Lys Gly Ser Gly Tyr Thr Phe Thr Asp Tyr
            20                  25                  30

Ala Met His Trp Val Lys Gln Ser Pro Gly Lys Ser Leu Glu Trp Ile
        35                  40                  45

Gly Val Ile Ser Thr Lys Tyr Gly Lys Thr Asn Tyr Asn Pro Ser Phe
    50                  55                  60

Gln Gly Gln Ala Thr Met Thr Val Asp Lys Ser Ser Ser Thr Ala Tyr
65                  70                  75                  80

Met Glu Leu Ala Ser Leu Lys Ala Ser Asp Ser Ala Ile Tyr Tyr Cys
                85                  90                  95

Ala Arg Gly Asp Glu Gly Tyr Ser Trp Gly Gln Gly Thr Ser Val Thr
            100                 105                 110

Val Ser Ser Ala Ser Thr Lys Gly Pro Ser Val Phe Pro Leu Ala Pro
        115                 120                 125

Cys Ser Arg Ser Thr Ser Glu Ser Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Lys Thr Tyr Thr Cys Asn Val Asp His Lys Pro Ser Asn Thr Lys
        195                 200                 205

Val Asp Lys Arg Val Glu Ser Lys Tyr Gly Pro Pro Cys Pro Pro Cys
    210                 215                 220

Pro Ala Pro Glu Phe Glu Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser Gln Glu Asp Pro Glu Val Gln Phe Asn Trp
            260                 265                 270

Tyr Val Asp Gly Val Glu Val His Asn Ala Lys Thr Lys Pro Arg Glu
        275                 280                 285

Glu Gln Phe Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Gly Leu Pro Ser Ser Ile Glu Lys Thr Ile Ser Lys Ala Lys Gly
                325                 330                 335

Gln Pro Arg Glu Pro Gln Val Tyr Thr Leu Pro Pro Ser Gln Glu Glu
            340                 345                 350

Met Thr Lys Asn Gln Val Ser Leu Thr Cys Leu Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Tyr Ser Arg Leu Thr Val Asp Lys Ser Arg Trp Gln Glu Gly Asn
                405                 410                 415

Val Phe Ser Cys Ser Val Met His Glu Ala Leu His Asn His Tyr Thr
            420                 425                 430

Gln Lys Ser Leu Ser Leu Ser Leu Gly
        435                 440

<210>  27
<211>  345
<212>  DNA
<213>  Artificial Sequence

<220>
<223>  VH2

<220>

<221>  CDS
<222>  (1)..(345)

<400>  27
gag gtc cag ctg cag cag tct ggg cct gag gtg gtg aag cct ggg gtc      48
Glu Val Gln Leu Gln Gln Ser Gly Pro Glu Val Val Lys Pro Gly Val
1               5                   10                  15

tca gtg aag att tcc tgc aag ggt tcc ggc tac aca ttc act gat tat      96
Ser Val Lys Ile Ser Cys Lys Gly Ser Gly Tyr Thr Phe Thr Asp Tyr
            20                  25                  30

gct atg cac tgg gtg aag cag agt cct ggc aag agt ctg gag tgg att      144
Ala Met His Trp Val Lys Gln Ser Pro Gly Lys Ser Leu Glu Trp Ile
        35                  40                  45

gga gtt att agt act aag tat ggt aag aca aac tac aac ccc agc ttt      192
Gly Val Ile Ser Thr Lys Tyr Gly Lys Thr Asn Tyr Asn Pro Ser Phe
    50                  55                  60

cag ggc cag gcc aca atg act gtt gac aaa tcc tcc agc aca gcc tat      240
Gln Gly Gln Ala Thr Met Thr Val Asp Lys Ser Ser Ser Thr Ala Tyr
65                  70                  75                  80

atg gag ctt gcc agc ttg aag gcc tcc gat tct gcc atc tat tac tgt      288
Met Glu Leu Ala Ser Leu Lys Ala Ser Asp Ser Ala Ile Tyr Tyr Cys
                85                  90                  95

gca aga ggg gac gag ggt tat tcc tgg ggt caa gga acc tca gtc acc      336
Ala Arg Gly Asp Glu Gly Tyr Ser Trp Gly Gln Gly Thr Ser Val Thr
            100                 105                 110

gtc tcc agc                                                          345
Val Ser Ser
        115


<210>  28
<211>  115
<212>  PRT
<213>  Artificial Sequence

<220>
<223>  Synthetic Construct

<400>  28

Glu Val Gln Leu Gln Gln Ser Gly Pro Glu Val Val Lys Pro Gly Val
1               5                   10                  15


Ser Val Lys Ile Ser Cys Lys Gly Ser Gly Tyr Thr Phe Thr Asp Tyr
            20                  25                  30


Ala Met His Trp Val Lys Gln Ser Pro Gly Lys Ser Leu Glu Trp Ile
        35                  40                  45


Gly Val Ile Ser Thr Lys Tyr Gly Lys Thr Asn Tyr Asn Pro Ser Phe
    50                  55                  60

Gln Gly Gln Ala Thr Met Thr Val Asp Lys Ser Ser Ser Thr Ala Tyr
65                  70                  75                  80

Met Glu Leu Ala Ser Leu Lys Ala Ser Asp Ser Ala Ile Tyr Tyr Cys
                85                  90                  95

Ala Arg Gly Asp Glu Gly Tyr Ser Trp Gly Gln Gly Thr Ser Val Thr
            100                 105                 110

Val Ser Ser
        115

<210>  29
<211>  18
<212>  DNA
<213>  Artificial Sequence

<220>
<223>  CDR3 VH2

<220>
<221>  CDS
<222>  (1)..(18)

<400>  29
ggg gac gag ggt tat tcc                                                18
Gly Asp Glu Gly Tyr Ser
1               5

<210>  30
<211>  6
<212>  PRT
<213>  Artificial Sequence

<220>
<223>  Synthetic Construct

<400>  30

Gly Asp Glu Gly Tyr Ser
1               5

<210>  31
<211>  48
<212>  DNA
<213>  Artificial Sequence

<220>
<223>  CDR1 VL2

<220>
<221>  CDS
<222>  (1)..(48)

<400>  31

aga tct ggt cag agc ctt gtg cac agt aat acc aac acc tat ctg cat     48
Arg Ser Gly Gln Ser Leu Val His Ser Asn Thr Asn Thr Tyr Leu His
1               5                   10                  15

<210>  32
<211>  16
<212>  PRT
<213>  Artificial Sequence

<220>
<223>  Synthetic Construct

<400>  32

Arg Ser Gly Gln Ser Leu Val His Ser Asn Thr Asn Thr Tyr Leu His
1               5                   10                  15

<210>  33
<211>  456
<212>  PRT
<213>  Artificial Sequence

<220>
<223>  Ab4 Heavy Chain

<400>  33

Gln Val Glu Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Asn Ala Ser Gly Thr Arg Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Lys Gly Asn Thr His Lys Pro Tyr Gly Tyr Val Arg Tyr
            100                 105                 110

Phe Asp Val Trp Gly Gln Gly Thr Leu Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Ser Asn Thr Lys Val Asp Lys Lys Val
    210                 215                 220

Glu Pro Lys Ser Cys Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350

Arg Glu Pro Gln Val Tyr Thr Leu Pro Pro Ser Arg Asp Glu Leu Thr
        355                 360                 365

Lys Asn Gln Val Ser Leu Thr Cys Leu Val Lys Gly Phe Tyr Pro Ser
    370                 375                 380

Asp Ile Ala Val Glu Trp Glu Ser Asn Gly Gln Pro Glu Asn Asn Tyr

385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro Gly Lys
    450                 455

<210>  34
<211>  215
<212>  PRT
<213>  Artificial Sequence

<220>
<223>  Ab4 Light Chain

<400>  34

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Val Pro Ala Arg Phe Ser
    50                  55                  60

Gly Ser Gly Ser Gly Thr Asp Phe Thr Leu Thr Ile Ser Ser Leu Glu
65                  70                  75                  80

Pro Glu Asp Phe Ala Thr Tyr Tyr Cys Leu Gln Ile Tyr Asn Met Pro
                85                  90                  95

Ile Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  35
<211>  454
<212>  PRT
<213>  Artificial Sequence

<220>
<223>  Ab5 Heavy chain

<400>  35

Glu Val Gln Leu Val Glu Ser Gly Gly Gly Leu Val Gln Pro Gly Gly
1               5                   10                  15

Ser Leu Arg Leu Ser Cys Ser Ala Ser Gly Phe Thr Phe Ser Ser Phe
            20                  25                  30

Gly Met His Trp Val Arg Gln Ala Pro Gly Lys Gly Leu Glu Trp Val
        35                  40                  45

Ala Tyr Ile Ser Ser Gly Ser Ser Thr Ile Tyr Tyr Gly Asp Thr Val
    50                  55                  60

Lys Gly Arg Phe Thr Ile Ser Arg Asp Asn Ala Lys Asn Ser Leu Phe
65                  70                  75                  80

Leu Gln Met Ser Ser Leu Arg Ala Glu Asp Thr Ala Val Tyr Tyr Cys
                85                  90                  95

Ala Arg Glu Gly Gly Tyr Tyr Tyr Gly Arg Ser Tyr Tyr Thr Met Asp
            100                 105                 110

Tyr Trp Gly Gln Gly Thr Thr Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Arg Val Glu Pro
    210                 215                 220

Lys Ser Cys Asp Lys Thr His Thr Cys Pro Pro Cys Pro Ala Pro Glu
225                 230                 235                 240

Leu Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser His Glu Asp Pro Glu Val Lys Phe Asn Trp Tyr Val Asp Gly
        275                 280                 285

Val Glu Val His Asn Ala Lys Thr Lys Pro Arg Glu Glu Gln Tyr Asn
    290                 295                 300

Ser Thr Tyr Arg Val Val Ser Val Leu Thr Val Leu His Gln Asp Trp
305                 310                 315                 320

Leu Asn Gly Lys Glu Tyr Lys Cys Lys Val Ser Asn Lys Ala Leu Pro
                325                 330                 335

Ala Pro Ile Glu Lys Thr Ile Ser Lys Ala Lys Gly Gln Pro Arg Glu
            340                 345                 350

Pro Gln Val Tyr Thr Leu Pro Pro Ser Arg Glu Glu Met Thr Lys Asn
        355                 360                 365

Gln Val Ser Leu Thr Cys Leu Val Lys Gly Phe Tyr Pro Ser Asp Ile
    370                 375                 380

Ala Val Glu Trp Glu Ser Asn Gly Gln Pro Glu Asn Asn Tyr Lys Thr
385                 390                 395                 400

Thr Pro Pro Val Leu Asp Ser Asp Gly Ser Phe Phe Leu Tyr Ser Lys
                405                 410                 415

Leu Thr Val Asp Lys Ser Arg Trp Gln Gln Gly Asn Val Phe Ser Cys
            420                 425                 430

Ser Val Met His Glu Ala Leu His Asn His Tyr Thr Gln Lys Ser Leu
        435                 440                 445

Ser Leu Ser Pro Gly Lys
    450

<210>  36
<211>  219
<212>  PRT
<213>  Artificial Sequence

<220>
<223>  Ab5 Light Chain

<400>  36

Asp Val Val Met Thr Gln Ser Pro Leu Ser Leu Pro Val Thr Pro Gly
1               5                   10                  15

Ala Pro Ala Ser Ile Ser Cys Arg Ser Ser Gln Ser Ile Val His Ser
            20                  25                  30

Asn Gly Asn Thr Tyr Leu Glu Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Arg Ile
65                  70                  75                  80

Ser Arg Val Glu Ala Glu Asp Val Gly Ile Tyr Tyr Cys Phe Gln Gly
                85                  90                  95

Ser His Val Pro Pro Thr Phe Gly Pro Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

## Description

### Field of the invention

The present invention relates to the prevention and/or treatment of Alzheimer's disease.

### Background

Alzheimer's disease is a complex multifactorial neurodegenerative disorder, which particularly results in loss of memory and impairment of cognitive functions. Neuropathological findings of Alzheimer's disease are characterized by the accumulation of extracellular deposits of beta-amyloid (Aβ) peptide in senile plaques and the intracellular aggregation of tau protein in neurofibrillary tangles. Alzheimer's disease (AD) progression is linked to an increased density of fibrillar Aβ plaques.

Document WO2010/130946 discloses humanized antibodies specific for the protofibrillar form of Aβ peptide and their use for the treatment of diseases associated with neurodegenerative disorders, such as Alzheimer's disease. The protofibrillar form of Aβ peptide disclosed in document WO2010/130946 corresponds to oligomers having a molecular weight greater than 200 kDa. Said humanized antibodies are characterized by a greatly improved affinity for the protofibrillar form of peptide Aβ by comparison to their affinity to the other forms of this peptide, which results in their specific binding to senile plaques and not to diffuse deposits.

Senile plaques are mainly composed of insoluble filamentous aggregates having a high molecular weight, the so-called fibrils, and of protofibrillar forms that have been shown to have a molecular weight greater than 200 kDa. On the contrary, diffuse deposits comprise no or little fibrils and protofibrillar forms of Aβ peptide. It is believed that diffuse plaques are precursors to senile plaques. Insoluble fibrils are indeed formed by the progressive aggregation of soluble Aβ peptides, which results in fibrillar forms that are initially soluble, i.e. protofibrillar forms, before to be converted into insoluble forms.

Soluble oligomers of Aβ peptide, including dimers, trimers and larger oligomers, are suggested to cause synaptic and cognitive dysfunction in the early stages of Alzheimer's disease. These soluble oligomers may be the major synaptotoxic Aβ species leading to neuronal dysfunction.

Alzheimer's disease has been recently reconceptualized as a progressive sequence of pathophysiological changes that includes asymptomatic, symptomatic predementia (mild cognitive impairment (MCI) due to AD or prodromal AD) and dementia phases. In the asymptomatic phase, biomarkers are used to establish the presence of AD pathology in subjects with no or very subtle overt symptoms.

There is still a need to provide solutions for the prevention of Alzheimer's disease, more particularly by intervention(s) initiated in cognitively unimpaired at-risk individuals to prevent the clinical onset of Alzheimer's disease and/or for the treatment of the earliest stages of the disease.

### Description of the invention

The Inventors have surprisingly found that antibodies specific for the protofibrillar form of the Aβ peptide, in particular specific for oligomers having a molecular weight greater than 200 kDa, have a neuronal cell protective effect against a preparation comprising several SDS-stable forms of Aβ peptide, which forms include small oligomers, large oligomers and aggregates having a molecular weight of less than 200 kDa.

Whereas these antibodies specific for the protofibrillar form of the Aβ peptide were previously disclosed as specifically binding to senile plaques and not to diffuse deposits, it was unexpected that these antibodies can prevent the neurotoxic effects of a Aβ preparation comprising several forms derived from synthetic Aβ42.

The Inventors have indeed shown that the humanized antibody Ab1 (consisting of two heavy chains of sequence SEQ ID NO: 2 and two light chains of sequence SEQ ID NO: 4) significantly inhibits the neurotoxic effects induced by a preparation comprising several SDS-stable forms of Aβ42 peptide (referred to as "oAβ42") in mouse primary neuronal cell cultures. Said humanized antibody indeed inhibits in a concentration-dependent manner both oAβ42-induced increase in capsase-3/7 enzymatic activity and oAβ42-induced decrease in neurite network.

The antibodies specific for the protofibrillar form of the Aβ peptide are thus particularly useful for the prevention of a disease involving Aβ peptide aggregation, in particular for the prevention of Alzheimer's disease, more particularly to prevent the clinical onset of Alzheimer's disease in a subject at risk of developing symptomatic Alzheimer's disease. These antibodies indeed allow preventing memory loss and impairment of cognitive functions. More particularly, they allow preventing memory loss and impairment of cognitive functions, which result from toxic Aβ oligomers and not from senile plaques.

The present invention thus relates to an antibody specific for the protofibrillar form of the Aβ peptide for use in the prevention of Alzheimer's disease and/or in the treatment of an early stage of symptomatic Alzheimer's disease.

The present invention also relates to a method for preventing Alzheimer's disease and/or treating an early stage of symptomatic Alzheimer's disease in a subject in need thereof, wherein said method comprises administering to said subject an effective amount of an antibody specific for the protofibrillar form of the Aβ peptide.

The present invention particularly relates to an antibody specific for the protofibrillar form of the Aβ peptide for use in the prevention of Alzheimer's disease in a subject (i) who does not suffer from memory loss and/or from impaired cognitive function and/or (ii) who does not have senile plaques, does not have neurodegeneration and/or does not have synapse dysfunction.

The antibody for use according to the invention preferably prevents, at least partially, Aβ-induced cytotoxicity in neuronal cells.

The antibody for use according to the invention preferably prevents at least partially, Aβ-induced apoptosis of neuronal cells and/or prevents, at least partially, Aβ-induced neurite network decrease.

The antibody is preferably a humanized antibody.

In one preferred embodiment, the antibody comprises (i) the CDRs of sequence SEQ ID NO: 10, 12, 14, 16, 18 and 20, (ii) the CDRs of sequence SEQ ID NO: 10, 12, 14, 32, 18 and 20 or (iii) the CDRs of sequence SEQ ID NO: 10, 12, 30, 32, 18 and 20.

The antibody preferably comprises the CDRs encoded by (i) the nucleotide sequences SEQ ID NO: 9, 11, 13, 15, 17 and 19, (ii) the nucleotide sequences SEQ ID NO: 9, 11, 13, 31, 17 and 19, (iii) the nucleotide sequences SEQ ID NO: 9, 11, 29, 31, 17 and 19 or (iv) by nucleotide sequences differing by respectively 1, 2, 3, 4 or 5 nucleotides from these sequences of (i), (ii) or (iii).

In one preferred embodiment, the variable part of the heavy chain of the antibody is encoded by a sequence having at least 80% identity with sequence SEQ ID NO: 5 or SEQ ID NO: 27 and/or (ii) the variable part of the light chain of the antibody is encoded by a sequence having at least 80% identity with sequence SEQ ID NO: 7 or SEQ ID NO: 23.

In one preferred embodiment, the variable part of the heavy chain of the antibody comprises a sequence having at least 80% identity with sequence SEQ ID NO: 6 or SEQ ID NO: 28 and/or the variable part of the light chain of the antibody comprises a sequence having at least 80% identity with sequence SEQ ID NO: 8 or SEQ ID NO: 24.

The antibody preferably comprises (i) a heavy chain encoded by a sequence having at least 80% identity with the nucleotide sequence SEQ ID NO: 1 or SEQ ID NO: 25 and/or (ii) a light chain encoded by a sequence having at least 80% identity with the nucleotide sequence SEQ ID NO: 3 or SEQ ID NO: 21.

The antibody preferably comprises (i) a heavy chain having at least 80% identity with the polypeptide sequence SEQ ID NO: 2 or SEQ ID NO: 26 and/or (ii) a light chain having at least 80% identity with the polypeptide sequence SEQ ID NO: 4 or SEQ ID NO: 22.

The antibody preferably comprises sequences encoded by the nucleotide sequences (i) SEQ ID NO: 5 and 7, (ii) the nucleotide sequences SEQ ID NO: 5 and 23 or (iii) the nucleotide sequences SEQ ID NO: 27 and 23.

The antibody is preferably characterized in that its sequence comprises (i) the polypeptide sequences SEQ ID NO: 6 and 8, (ii) the polypeptide sequences SEQ ID NO: 6 and 24 or (iii) the polypeptide sequences SEQ ID NO: 28 and 24.

The antibody preferably comprises the sequences encoded by (i) the nucleotide sequences SEQ ID NO: 1 and 3, (ii) the nucleotide sequences SEQ ID NO: 1 and 21 or (iii) the nucleotide sequences SEQ ID NO: 25 and 21.

The antibody preferably comprises (i) the polypeptide sequences SEQ ID NO: 2 and 4, (ii) the polypeptide sequences SEQ ID NO: 2 and 22 or (iii) the polypeptide sequences SEQ ID NO: 26 and 22.

### Aβ peptide and aggregated forms thereof

By the expression "Aβ peptide" or "amyloid beta peptide" or "Abeta peptide", it is herein meant a peptide of 36 to 43 amino acids resulting from sequential cleavage of the amyloid precursor protein (APP) by beta secretase and gamma secretase.

The Aβ42 peptide is the more hydrophobic and fibrillogenic peptide and constitutes the main species deposited in the brain.

Aβ peptides can be found as monomers or can aggregate to form soluble oligomers or insoluble fibrils.

A soluble oligomer is for example a dimer, trimer, tetramer, larger oligomer or a protofibrillar form of Aβ peptide.

By the expression "protofibrillar form", it is herein meant an oligomeric form of Aβ peptides, which is soluble *in vitro,* which can be isolated as an entity of molecular weight greater than 200 kDa, 300 kDa, 400 kDa or 500 kDa and which can fix agents such as thioflavin-S or Congo Red.

By the expression "senile plaque", it is herein meant a plaque composed of an amyloid core (fixing thioflavin S or Congo Red) surrounded by dystrophic neurites and a reaction of glial cells. *In vivo,* senile plaques mainly comprise insoluble fibrils and protofibrillar forms having a molecular weight greater than 200 kDa. Senile plaques are found in particular in patients with Alzheimer's disease.

By the expression "diffuse amyloid deposit" our "diffuse deposit", it is herein meant amorphous Aβ substructure that do not fix thioflavin S or Congo Red and comprise no or little fibrils and protofibrillar forms. Diffuse amyloid deposits are far more numerous, but are not associated with the disease.

### Subject to be treated

A subject in need to be treated for the prevention and/or the treatment of Alzheimer's disease according to the invention may be a human being or a non-human mammal, preferably a human being.

When the subject is a human being, the terms "individual" or "patient" may also be used.

Said human being may be of any age, for example an infant, child, adolescent, adult, elderly people.

A non-human mammal is preferably a mouse, rat, cat, dog, rabbit or primate.

The subject may be a subject at risk to develop symptomatic Alzheimer's disease, in particular for the prevention of Alzheimer's disease, or a subject at an early stage of symptomatic Alzheimer's disease, in particular for the treatment of symptomatic Alzheimer's disease.

A subject at risk to develop symptomatic Alzheimer's disease does not suffer from memory loss and does not suffer from impairment of cognitive functions. In other words, a subject at risk to develop symptomatic Alzheimer's disease has normal cognition or optionally subtle cognitive decline.

Memory loss may be assessed by any method well-known by the skilled person, such as but not limited to, the Free and Cued Selective Reminding Test, the Rey Auditory Verbal Learning Test and/or the California Verbal Learning Test.

Impairment of cognitive functions may be assessed by any method well-known by the skilled person, such as but not limited to, the Mini-Mental State Examination, the Alzheimer's Disease Assessment Scale -Cognitive Subscale, the Neurospychological Test Battery and/or the Repeatable Battery for the Assessment of Neuropsychological Status.

A subject at risk to develop symptomatic Alzheimer's disease may be a pre-Alzheimer subject or a subject with asymptomatic Alzheimer's disease.

A pre-Alzheimer subject does not have any biomarker associated with a risk of developing symptomatic Alzheimer's disease.

A subject with asymptomatic Alzheimer's disease has at least one biomarker and/or at least one genetic factor associated with a risk of developing symptomatic Alzheimer's disease.

Such biomarkers associated with a risk of developing symptomatic Alzheimer's disease include biomarkers of Aβ peptide accumulation in the brain (for example, abnormal tracer retention on amyloid PET imaging and/or low levels of Aβ peptide in cerebrospinal fluid (CSF)) and/or biomarkers of neuronal degeneration or injury (for example elevated levels of tau protein in CSF and/or decreased brain fluorodeoxyglucose uptake on PET imaging and/or brain atrophy on structural magnetic resonance).

Such genetic factors associated with a risk of developing symptomatic Alzheimer's disease include, but are not limited to,
- for autosomal dominant Alzheimer's disease, mutation(s) in APP, PSEN1 and/or PSEN2 genes;
- for sporadic Alzheimer's disease, mutation(s) of gene(s) involved in lipid metabolism (APOE4, CLU and/or ABCA7), in the immune system (CLU, CR1, ABCA7, CD33, EPHA1 and/or the MS4A gene cluster), and/or in synaptic functioning (PICALM, CD33, CD2AP, EPHA1 and/or BIN1).

Memory loss and impairment of cognitive functions result, at least partially, from synapse dysfunction and/or neurodegeneration.

Thus, a subject at risk to develop symptomatic Alzheimer's disease does not have synapse dysfunction and/or neurodegeneration or has a level of synapse dysfunction and/or neurodegeneration not translating into symptoms.

By the expression "synapse dysfunction", it is herein meant abnormal alterations in the number, structure and/or organization of synapses, in particular in neurodegenerative conditions, leading to impairment in the functional activity of synapses. Synapse dysfunction includes mechanisms of synapse loss.

Synapse dysfunction may be assessed by any method well-known by the skilled person, such as for example assessment of functional brain connectivity using resting state- or task-functional MRI (*Magnetic Resonance Imaging*) (*see for example* Knight et al. 2016, Magnetic Resonance Imaging to Detect Early Molecular and Cellular Changes in Alzheimer's Disease, Front. Aging Neurosci. Vol. 8, article 139*)* and/or measurement of levels of synaptic protein(s) (for example, neurogranin) in CSF.

Synapse dysfunction, as observed *in vivo,* for example corresponds *in vitro* to impaired neurite network.

By the expression "impaired neurite network", it is herein meant a decreased neurite network, i.e. decrease in the size of neurite length.

Neurite network may be assessed by any method well-known by the skilled person, such as immunocytochemistry followed by cell imaging.

By the expression "neurodegeneration", it is herein meant loss of neuronal cells and/or decrease in neuronal function or activity.

Neurodegeneration may be assessed by any method well-known by the skilled person, such as for example elevated levels of tau protein in CSF and/or decreased brain fluorodeoxyglucose uptake on PET imaging and/or brain atrophy on structural magnetic resonance.

Neurodegeneration, as observed *in vivo,* for example corresponds *in vitro* to abnormal neuronal cell apoptosis.

Abnormal neuronal cell apoptosis means an increased neuronal cell apoptosis by comparison to a control healthy cell.

Neuronal cell apoptosis may be assessed by any method well-known by the skilled person, such as measurement of enzymatic activity of caspase 3/7.

In one preferred embodiment of the invention, a subject at risk to develop symptomatic Alzheimer's disease is:
- a subject with normal cognition having at least one family member that is suffering from or is at risk to suffer from symptomatic Alzheimer's disease, more preferably from an autosomal dominant form of Alzheimer's disease, and/or
- a subject with normal cognition diagnosed as carrying at least one mutation involved in an autosomal dominant form of Alzheimer's disease, and/or
- a subject with normal cognition or with subtle cognitive decline, at risk of developing symptomatic Alzheimer's disease on the basis of biomarker(s) and/or genetic factor(s) associated with a risk of developing symptomatic Alzheimer's disease, this subject being for example at risk of developing a sporadic form of Alzheimer's disease.

Said biomarker(s) and/or genetic factor(s) associated with a risk of developing symptomatic Alzheimer's disease are as defined above.

By the expression "family member", it is preferably meant a member of previous generations (for example, parent, grand-parent or great-grant parent), a brother, a sister or optionally a child or grandchild. A family member is generally a parent or sibling.

By the expression "autosomal dominant form of Alzheimer's disease", it herein meant an Alzheimer's disease resulting from at least one genetic mutation.

Autosomal dominant forms of Alzheimer's disease are well-known by the skilled person. Autosomal dominant forms of Alzheimer's disease for example include at least one mutation in at least one gene selected in the group consisting of a gene encoding Amyloid Beta Precursor Protein (APP), a gene encoding Presenilin 1 (PSEN1), and a gene encoding Presenilin 2 (PSEN2).

Non-limitative examples of such mutations are described by Cacace et al. (2016, Alzheimer's & Dementia vol.12, pp 733-748).

A subject having developed symptomatic Alzheimer's disease, i.e. suffering from symptomatic Alzheimer's disease, is preferably at an early stage of symptomatic Alzheimer's disease.

By "early stage of symptomatic Alzheimer's disease", it is herein meant a stage wherein memory loss and/or impairment of cognitive functions begin.

An example of early stage of symptomatic Alzheimer's disease is symptomatic predementia.

Symptomatic predementia for example includes mild cognitive impairment (MCI) due to Alzheimer's disease or prodromal Alzheimer's disease. Characteristics of MCI due to Alzheimer's disease and of prodromal Alzheimer's disease may be found in Albert et al., 2011, Alzheimer's & Dementia, 7, 270-279 and Dubois et al., 2016, Alzheimer's & Dementia, 12, 292-323, respectively.

A later stage of symptomatic Alzheimer's disease is dementia due to Alzheimer's disease, preferably mild dementia due to Alzheimer's disease. Characteristics of dementia due to Alzheimer's disease may be found in McKhann, 2011, Alzheimer's & Dementia, 7, 263269.

In one preferred embodiment of the invention, the subject does not suffer from symptomatic Alzheimer's disease.

In a preferred embodiment of the invention, the subject does not have senile plaques.

The absence of senile plaque may for example be assessed by amyloid PET *(Positron Emission Tomography)* imaging of the brain.

### Prevention of Alzheimer's disease or treatment of an early stage of symptomatic Alzheimer's disease

By the expression "prevention of Alzheimer's disease", it is herein meant to prevent, at least partially, the memory loss and/or impairment of cognitive function in a subject to be treated, preferably in a subject at risk to develop symptomatic Alzheimer's disease, in particular by preventing, at least partially, Aβ-induced cytotoxicity in neuronal cells.

Desirable effects of the prevention of Alzheimer's disease include:
- preventing, at least partially, neurodegeneration, , and/or
- preventing, at least partially, synaptic dysfunction, in particular preventing, at least partially, the loss of synapse.

By the expression "treatment of an early stage of symptomatic Alzheimer's disease", it is herein meant to treat, at least partially, the memory loss and/or impairment of cognitive function in a subject to be treated, preferably in a subject suffering from an early stage of symptomatic Alzheimer's disease, for example a subject suffering from symptomatic predementia, in particular by preventing, at least partially, Aβ-inducted cytotoxicity in neuronal cells.

Desirable effects of treatment of an early stage of symptomatic Alzheimer's disease include:
- stopping or slowing down neurodegeneration and/or
- stopping or slowing down the development of synaptic dysfunction, in particular stopping or slowing down the loss of synapse.

The expressions "memory loss", "impairment of cognitive function" and "subject to be treated" are as defined above.

Neurodegeneration, synapse dysfunction and synapse loss are as defined above and may be assessed as described above.

Memory loss and impairment of cognitive functions to be prevented and/or treated according to the invention result from toxic Aβ oligomers, and not from senile plaques.

### Antibody

Naturally occurring antibodies typically comprise a tetramer. Each such tetramer is typically composed of two identical pairs of polypeptide chains, each pair having one full-length "light" chain (typically having a molecular weight of about 25 kDa) and one full-length "heavy" chain (typically having a molecular weight of about 50-70 kDa). The terms "heavy chain" and "light chain" as used herein refer to any immunoglobulin polypeptide having sufficient variable domain sequence to confer specificity for a target antigen. The amino-terminal portion of each light and heavy chain typically includes a variable domain of about 100 to 110 or more amino acids that typically is responsible for antigen recognition. The carboxy-terminal portion of each chain typically defines a constant domain responsible for effector function. Thus, in a naturally occurring antibody, a full-length heavy chain immunoglobulin polypeptide includes a variable domain (VH) and three constant domains (CH1, CH2, and CH3), wherein the VH domain is at the amino-terminus of the polypeptide and the CH3 domain is at the carboxyl-terminus, and a full-length light chain immunoglobulin polypeptide includes a variable domain (VL) and a constant domain (CL), wherein the VL domain is at the amino-terminus of the polypeptide and the CL domain is at the carboxyl-terminus.

Human light chains are typically classified as kappa and lambda light chains, and human heavy chains are typically classified as mu, delta, gamma, alpha, or epsilon, and define the antibody's isotype as IgM, IgD, IgG, IgA, and IgE, respectively. IgG has several subclasses, including, but not limited to, IgG1, IgG2, IgG3, and IgG4. IgM has subclasses including, but not limited to, IgM1 and IgM2. IgA is similarly subdivided into subclasses including, but not limited to, IgA1 and IgA2. Within full-length light and heavy chains, the variable and constant domains typically are joined by a "J" region of about 12 or more amino acids, with the heavy chain also including a "D" region of about 10 more amino acids. See, e.g., FUNDAMENTAL IMMUNOLOGY (Paul, W., ed., Raven Press, 2nd ed., 1989). The variable regions of each light/heavy chain pair typically form an antigen binding site. The variable domains of naturally occurring antibodies typically exhibit the same general structure of relatively conserved framework regions (FR) joined by three hypervariable regions, also called complementarity determining regions or CDRs. The CDRs from the two chains of each pair typically are aligned by the framework regions, which may enable binding to a specific epitope. From the amino-terminus to the carboxyl-terminus, both light and heavy chain variable domains typically comprise the domains FR1, CDR1, FR2, CDR2, FR3, CDR3 and FR4.

The term "CDR set" refers to a group of three CDRs that occur in a single variable region capable of binding the antigen. The exact boundaries of these CDRs have been defined differently according to different systems. The system described by Kabat (Kabat et al., SEQUENCES OF PROTEINS OF IMMUNOLOGICAL INTEREST (National Institutes of Health, Bethesda, Md. (1987) and (1991)) not only provides an unambiguous residue numbering system applicable to any variable region of an antibody, but also provides precise residue boundaries defining the three CDRs. These CDRs may be referred to as Kabat CDRs. Chothia and coworkers (Chothia and Lesk, 1987, J. M01. Biol. 196: 901-17; Chothia et al., 1989, Nature 342: 877-83) found that certain sub-portions within Kabat CDRs adopt nearly identical peptide backbone conformations, despite having great diversity at the level of amino acid sequence. These sub-portions were designated as L1, L2, and L3 or H1, H2, and H3 where the "L" and the "H" designates the light chain and the heavy chain regions, respectively. These regions may be referred to as Chothia CDRs, which have boundaries that overlap with Kabat CDRs. Other boundaries defining CDRs overlapping with the Kabat CDRs have been described by Padlan, 1995, FASEB J. 9: 133- 39; MacCallum, 1996, J. Mol. Biol. 262(5): 732-45; and Lefranc, 2003, Dev. Comp. Immunol. 27: 55-77. Still other CDR boundary definitions may not strictly follow one of the herein systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although certain embodiments use Kabat or Chothia defined CDRs. Identification of predicted CDRs using the amino acid sequence is well known in the field, such as in Martin, A.C. "Protein sequence and structure analysis of antibody variable domains," In Antibody Engineering, Vol. 2. Kontermann R., Dubel S., eds. Springer-Verlag, Berlin, p. 33-51 (2010). The amino acid sequence of the heavy and/or light chain variable domain may be also inspected to identify the sequences of the CDRs by other conventional methods, e.g., by comparison to known amino acid sequences of other heavy and light chain variable regions to determine the regions of sequence hypervariability. The numbered sequences may be aligned by eye, or by employing an alignment program such as one of the CLUSTAL suite of programs, as described in Thompson, 1994, Nucleic Acids Res. 22: 4673-80. Molecular models are conventionally used to correctly delineate framework and CDR regions and thus correct the sequence-based assignments.

The term "antibody" as used herein encompasses an antibody as defined above and any functional fragment of antibody. Preferably, the functional fragment of an antibody is a fragment of type Fv, scFv (*single chain Fv*), Fab (*Fragment antigen binding*), F(ab')2, Fab', scFv-Fc, diabody, multispecific antibody (notably bispecific), synthetic polypeptide containing the sequence of one or more CDRs, which generally possesses the same specificity of fixation as the antibody from which they are derived. Functional fragments of an antibody can be obtained by methods such as digestion by enzymes, such as pepsin or papain and/or by cleavage of the disulphide bridges by chemical reduction. Nanobodies also come under the definition of an antibody.

The term "Fc" as used herein refers to a molecule comprising the sequence of a non-antigen-binding fragment resulting from digestion of an antibody or produced by other means, whether in monomeric or multimeric form, and can contain the hinge region. The original immunoglobulin source of the native Fc is preferably of human origin and can be any of the immunoglobulins, although IgG1 and IgG2 are preferred. Fc molecules are made up of monomeric polypeptides that can be linked into dimeric or multimeric forms by covalent (i.e., disulfide bonds) and non-covalent association. The number of intermolecular disulfide bonds between monomeric subunits of native Fc molecules ranges from 1 to 4 depending on class (e.g., IgG, IgA, and IgE) or subclass (e.g., IgG1, IgG2, IgG3, IgA1, and IgGA2). One example of a Fc is a disulfide-bonded dimer resulting from papain digestion of an IgG. The term "Fc" as used herein is generic to the monomeric, dimeric, and multimeric forms.

A F(ab) fragment typically includes one light chain and the VH and CH1 domains of one heavy chain, wherein the VH-CH1 heavy chain portion of the F(ab) fragment cannot form a disulfide bond with another heavy chain polypeptide. As used herein, a F(ab) fragment can also include one light chain containing two variable domains separated by an amino acid linker and one heavy chain containing two variable domains separated by an amino acid linker and a CH1 domain.

A F(ab') fragment typically includes one light chain and a portion of one heavy chain that contains more of the constant region (between the CH1 and CH2 domains), such that an interchain disulfide bond can be formed between two heavy chains to form a F(ab')2 molecule.

"Epitope" means the site of the antigen to which the antibody binds. If the antigen is a polymer, such as a protein or a polysaccharide, the epitope can be formed by contiguous or non-contiguous residues. Here the epitope is conformational, i.e. related to the three-dimensional structure of the protofibrillar A-β peptide.

The term "VH" as used herein refers to the variable region of a heavy chain of an antibody, including the heavy chain of a fragment Fv, scFv, dsFv, Fab, Fab' or F(ab)'.

The term "VL" as used herein refers to the variable region of a light chain of an antibody, including the light chain of a fragment Fv, scFv, dsFv, Fab, Fab' or F(ab)'.

"Humanized antibody" means an antibody that contains mainly human immunoglobulin sequences. This term generally refers to a non-human immunoglobulin that has been modified by incorporating human sequences or residues found in human sequences.

In general, humanized antibodies comprise one or typically two variable domains in which all or part of the CDR regions correspond to parts derived from the non-human parent sequence and in which all or part of the FR regions are derived from a human immunoglobulin sequence. The humanized antibody can then comprise at least one portion of a constant region of immunoglobulin (Fc), in particular that of the selected reference human immunoglobulin.

A humanized antibody is preferably the least immunogenic as possible in a human. For that purpose, it is possible that one or two amino acids of one or more CDRs are modified by an amino acid that is less immunogenic for the human host, without substantially reducing the binding specificity of the antibody to the protofibrillar form of the Aβ peptide. Furthermore, the residues of the framework regions do not need to be human and it is possible that they are not modified, as they do not contribute to the immunogenic potential of the antibody.

Several methods of humanization are known by a person skilled in the art for modifying a non-human parent antibody to an antibody that is less immunogenic in humans. Complete identity of the sequences with a human antibody is not essential. In fact complete sequence identity is not necessarily a predictive indicator of reduced immunogenicity and modification of a limited number of residues can lead to humanized antibodies presenting a very attenuated immunogenic potential in humans (Molecular Immunology (2007) 44, 1986-1998).

Some methods are for example the inclusion of CDRs (grafting) (EPO 0 239 400; WO 91/09967; and U.S. Pat. Nos. 5,530,101 and 5,585,089), the resurfacing (EPO 0 592 106; EPO 0 519 596; Padlan, 1991, Molec Imm 28(4/5): 489-498; Studnicka et al., 1994, Prot Eng 7(6):805-814; and Roguska et al., 1994, PNAS 91:969-973) or chain mixing (U.S. Pat. No. 5,565,332).

The humanized antibody may have variable parts that are modified according to the technology explained in international application WO 2009/032661.

This technique notably uses dynamic molecular simulation based on three-dimensional models of antibodies, said models being constructed by homology.

The antibody may have diminished effector functions, such as immunoglobulins bearing mutations of the Fc domain reducing its affinity for the receptors of the immune system or such as nanobodies.

"Effector functions" means any fixation of the Fc domain of the antibody to receptors or proteins inducing immune responses. Decreasing these effector functions makes it possible to reduce adverse effects such as the induction of microhaemorrhages (Racke et al. J Neurosci 2005, 25:629).

Affinity can be measured by any technique known by a person skilled in the art. It is advantageously measured by the Biostat Speed technique developed on the basis of the algorithms described by Ratkovsk D A and Reedy T J (Bio-metrics, 1986, 42, 575-82).

In order to express the heavy chain(s) and/or light chain(s) of an antibody, the polynucleotides coding for said chain(s) may be inserted in expression vector(s). The expression vector can be a plasmid, YAC, cosmid, retrovirus, episome derived from EBV, or any vector that a person skilled in the art may judge suitable for expression of said chain(s).

The vector can be used for transforming cells, advantageously derived from one cell line. Said cell line is even more advantageously derived from a mammal. It is advantageously the CHO line or a line derived from this line, or the HEK293 line or a line derived from this line.

The transformation of the cells can be carried out by any method well-known by a person skilled in the art for introducing polynucleotides into a host cell. Said method can be transformation by means of dextran, precipitation by calcium phosphate, transfection by means of polybrene, protoplast fusion, electroporation, encapsulation of the polynucleotides in liposomes, biolistic injection and direct micro-injection of DNA into the nucleus.

### Sequence identity

By "an amino acid or nucleic acid sequence at least x% identical to a reference sequence", it is intended that the sequence of the subject peptide or nucleic acid is identical to the reference sequence or differ from the reference sequence by up to 100-x amino acid or nucleotide alterations per each 100 amino acids or nucleotides of the reference sequence. In other words, to obtain a polypeptide or nucleic acid having a sequence at least x% identical to a reference sequence, up to 100-x% of the amino acids or nucleotides in the subject sequence may be inserted, deleted or substituted with another amino acid or nucleotide.

A polypeptide having at least 80%, 85%, 90%, 95% or 99% identity with a reference sequence may comprise mutation(s), such as deletion(s), insertion(s) and/or substitution(s) compared to the reference sequence. In case of substitutions, the substitution preferably corresponds to a conservative substitution as indicated in the Table 1 below and/or to a substitution for a less immunogenic amino acid, more particularly for a human host.

A nucleic acid sequence "at least 80%, 85%, 90%, 95% or 99% identical" to a reference sequence may comprise mutation(s), such as deletion(s), insertion(s) and/or substitution(s) compared to the reference sequence. In case of substitution, the substitution preferably corresponds to a silent substitution or a substitution leading to a conservative substitution in the translated amino acid sequence or to a less immunogenic amino acid, by comparison to the reference sequence, for example as indicated in the Table 1 above.

**Table 1**

| **Conservative substitutions** | **Type of Amino Acid** |
|---|---|
| Ala, Val, Leu, Ile, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

In a preferred embodiment, the nucleic sequence at least 80%, 85%, 90%, 95% or 99% identical to a reference sequence only differs from the reference sequence by silent substitution(s) and/or substitution(s) leading to a conservative amino-acid substitution and/or to a less immunogenic amino acid.

Methods for comparing the identity of two or more sequences are well known in the art. For instance, programs available in the Wisconsin Sequence Analysis Package, version 9.1, for example the programs BESTFIT and GAP, may be used to determine the % identity between two sequences. BESTFIT uses the "local homology" algorithm of Smith and Waterman and finds the best single region of similarity between two sequences. Other programs for determining identity between sequences are also known in the art, for instance the Needle program, which is based on the Needleman and Wunsch algorithm, described in Needleman and Wunsch (1970) J. Mol Biol. 48:443-453, with for example the following parameters for polypeptide sequence comparison: comparison matrix: BLOSUM62, gap open penalty: 10 and gap extend penalty: 0.5, end gap penalty: false, end gap open penalty = 10, end gap extend penalty = 0.5; and the following parameters for polynucleotide sequence comparison: comparison matrix: DNAFULL; gap open penalty = 10, gap extend penalty = 0.5, end gap penalty: false, end gap open penalty = 10, end gap extend penalty = 0.5.

### Antibody specific for the protofibrillar form of the Aβ peptide

The antibody for use according to the invention is specific for the protofibrillar form of the Aβ peptide.

The antibody thus specifically binds to the protofibrillar form of the Aβ peptide, i.e. to a high molecular weight peptide, which is as defined above. The antibody particularly binds to the Aβ peptide having a molecular weight greater than 200, 300, 400 or 500 kDa.

The antibody preferably binds specifically to the protofibrillar form of the Aβ peptide but not to the other proteins of amyloid structure (for example IAPP, Islet Amyloid Polypeptide).

By "specific binding", it is herein meant a difference by a factor of at least about 10, 20, 30, 40, 50, or 100 between the strength of binding to the protofibrillar form of the Aβ peptide relative to the binding to the other forms of this peptide.

The antibody preferably has an affinity for the protofibrillar form of peptide Aβ at least 100 times greater, more preferably at least 150 times greater, still more preferably at least 170 times greater than its affinity for the other forms of this peptide. The affinity may be the EC50 measured by ELISA.

The antibody is thus characterized by its ability to bind to Aβ peptides aggregated into protofibrills.

The antibody for use according to the invention also prevents, at least partially, Aβ-induced apoptosis of neuronal cells and/or prevents, at least partially, Aβ-induced neurite network decrease, in particular *in vitro.*

Neuronal cell apoptosis and neurite network may be assessed as described above or in the Examples.

The antibody is preferably a humanized antibody.

The humanized antibody preferably has reduced effector functions, making it possible to limit adverse effects such as the development of microhemorrhages and/or vasogenic edemas.

A preferred antibody for use according to the invention no longer possesses effector functions.

Advantageously, the antibody may be an immunoglobulin G4 whose Fc domain has undergone mutations reducing the production of half-molecules.

Advantageously, the antibody may be an immunoglobulin G4 whose Fc domain has undergone mutations reducing the effector activity.

The antibody preferably comprises at least one CDR encoded by a nucleotide sequence having a sequence identical to one of the sequences SEQ ID NO: 9, 11, 13, 15, 17 and 19, or by sequences differing respectively by 1, 2, 3, 4 or 5 nucleotides from these sequences.

The antibody preferably comprises at least one CDR having a sequence identical to one of the sequences SEQ ID NO: 10, 12, 14, 16, 18 and 20.

The antibody preferably comprises at least one CDR whose sequence differs by one to two amino acids relative to one of the sequences SEQ ID NO: 10, 12, 14, 16, 18, 20 and 32, inasmuch as the antibody maintains its binding specificity.

A preferred antibody comprises the CDRs of sequence SEQ ID NO: 10, 12, 14, 16, 18 and 20, the CDRs of sequence SEQ ID NO: 10, 12, 14, 32, 18 and 20, or the CDRs of sequence SEQ ID NO: 10, 12, 30, 32, 18 and 20.

The antibody for example comprises the CDRs encoded by (i) the nucleotide sequences SEQ ID NO: 9, 11, 13, 15, 17 and 19, (ii) the nucleotide sequences SEQ ID NO: 9, 11, 13, 31, 17 and 19, (iii) the nucleotide sequences SEQ ID NO: 9, 11, 29, 31, 17 and 19 or (iv) by sequences differing respectively by 0, 1, 2, 3, 4 or 5 nucleotides from these sequences of (i), (ii) or (iii).

In one preferred embodiment, the antibody is a humanized antibody:
- comprising the CDRs of sequence SEQ ID NO: 10, 12, 14, 16, 18 and 20, the CDRs of sequence SEQ ID NO: 10, 12, 14, 32, 18 and 20 or the CDRs of sequence SEQ ID NO: 10, 12, 30, 32, 18 and 20, and
- having an affinity for the protofibrillar form of the Aβ peptide being at least 100 times greater than its affinity for the other forms of this peptide, the affinity being preferably the EC50 measured by ELISA.

In one advantageous embodiment, the antibody comprises or consists of (i) a variable part of its heavy chain (VH) encoded by a sequence having at least 80%, 85%, 90%, 95% or 99% identity with the sequence SEQ ID NO: 5 or the sequence SEQ ID NO: 27 and/or (ii) a variable part of its light chain (VL) encoded by a sequence having at least 80%, 85%, 90%, 95% or 99% identity with the sequence SEQ ID NO: 7 or the sequence SEQ ID NO: 23.

In one advantageous embodiment, the antibody comprises or consists of (i) a variable part of its heavy chain (VH) comprising a sequence having at least 80%, 85%, 90%, 95% or 99% identity with the sequence SEQ ID NO: 6 or the sequence SEQ ID NO: 28 and/or (ii) a variable part of its light chain (VL) comprising a sequence having at least 80%, 85%, 90%, 95% or 99% identity with the sequence SEQ ID NO: 8 or the sequence SEQ ID NO: 24.

In one even more advantageous embodiment, the humanized antibody comprises or consists of (i) a heavy chain comprising a variable part (VH) encoded by the nucleotide sequences SEQ ID NO: 5 or SEQ ID NO 27 and/or (ii) a light chain comprising a variable part (VL) encoded by the nucleotide sequences SEQ ID NO 7 or SEQ ID NO 23.

In one even more advantageous embodiment, the humanized antibody comprises or consists of (i) a heavy chain comprising a variable part (VH) of polypeptide sequence SEQ ID NO: 6 or SEQ ID NO: 28 and/or (ii) a light chain comprising a variable part (VL) of polypeptide sequence SEQ ID NO 8 or SEQ ID NO 24.

In one advantageous embodiment, the humanized antibody comprises or consists of the polypeptide sequences encoded by (i) the nucleotide sequences SEQ ID NO: 5 and 7, (ii) the nucleotide sequences SEQ ID NO: 5 and 23 or (iii) the nucleotide sequences SEQ ID NO: 27 and 23.

In one advantageous embodiment, the humanized antibody comprises or consists of (i) the polypeptide sequences SEQ ID NO: 6 and 8, (ii) the polypeptide sequences SEQ ID NO: 6 and 24, or (iii) the polypeptide sequences SEQ ID NO: 28 and 24.

The antibody may comprise or consists of (i) a heavy chain encoded by a sequence having at least 80%, 85%, 90%, 95% or 99% identity with the nucleotide sequence SEQ ID NO: 1 or SEQ ID NO: 25, and/or (ii) a light chain encoded by a sequence having at least 80%, 85%, 90%, 95% or 99% identity with the nucleotide sequence SEQ ID NO: 3 or SEQ ID NO: 21.

The antibody may comprise or consists of (i) a heavy chain having at least 80%, 85%, 90%, 95% or 99% identity with the polypeptide sequence SEQ ID NO: 2 or with the polypeptide sequence SEQ ID NO: 26, and/or (ii) a light chain comprising a sequence having at least 80%, 85%, 90%, 95% or 99% identity with the polypeptide sequence SEQ ID NO: 4 or SEQ ID NO: 22.

The humanized antibody for example comprises or consists of the sequences encoded by the nucleotide sequences SEQ ID NO: 1 and 3, the nucleotide sequences SEQ ID NO: 1 and 21, or the nucleotide sequences SEQ ID NO: 25 and 21.

The humanized antibody for example comprises or consists of the polypeptide sequences SEQ ID NO: 2 and 4, the polypeptide sequences SEQ ID NO: 2 and 22 or the polypeptide sequences SEQ ID NO: 26 and 22.

The humanized antibody, for example antibody Ab1 (VH1 + VL1), Ab2 (VH1 + VL2) or Ab3 (VH2+VL2) may be produced by transient expression in a mammalian line, for example line HEK293 designated FreeStyle 293-F.

The cDNAs coding for the humanized variable domain of the light chain and heavy chain (for example, VL1 or VL2; and VH1 or VH2) are fused with the cDNAs coding for the human constant regions, for example Ckappa and IgG4, respectively. The sequence of the constant region IgG4 may be the sequence of the variant having the substitutions S241 P and L248E in Kabat's nomenclature, for a significant reduction in the production of half-molecules (*see for example* Angla et al., 1993, Mol. Immunol., 30: 105-108) and the effector functions (*see for example* WO 97/09351).

The nucleic acid sequences coding for the heavy chain (for example VH1+CH1 or VH2 + CH2) and for the light chain (for example VL1 + CL1 or VL2 + CL2), respectively, may be cloned independently in two expression vectors. The humanized antibody is then produced by transient expression in a mammalian cell, after co-transfection with these two expression vectors. The antibodies are then preferably purified by affinity chromatography, for example on a column of MabSelect gel (Amersham), preferably formulated in a buffer, for example a PBS buffer, and optionally submitted to sterile filtration (0.2 µm).

### Pharmaceutical composition

The antibody specific for the protofibrillar form of the Aβ peptide for use according to the invention is preferably provided in a pharmaceutical composition.

The term "pharmaceutical composition" as used herein refers to a composition capable of inducing a desired therapeutic effect, when properly administered to a subject.

The pharmaceutical composition comprises an antibody specific for the protofibrillar form of the Aβ peptide as defined above and at least one pharmaceutically acceptable carrier.

The expression "pharmaceutically acceptable carrier" or "physiologically acceptable carrier" as used herein refers to one or more formulation materials suitable for accomplishing or enhancing the delivery of an antibody.

The pharmaceutical composition preferably comprises at least one pharmaceutically acceptable vehicle for an injectable formulation. Such vehicles are well known by the skilled person and include sterile, isotonic saline solutions (monosodium or disodium phosphate, sodium, potassium, calcium or magnesium chloride, and/or mixtures of said salts), or dry compositions, notably lyophilized, which, by adding sterilized water or physiological serum as appropriate, permit injectable salutes to be constituted.

As an example, a pharmaceutical composition comprises (1) a Dulbecco phosphate buffer (pH ∼7.4), optionally containing 1mg/ml to 25 mg/ml of human serum albumin, (2) 0.9% w/v of sodium chloride (NaCl), and (3) 5% (w/v) of dextrose. It can also comprise an antioxidant such as tryptamine and a stabilizer such as Tween 20.

The pharmaceutical composition preferably comprises an effective amount of the antibody specific for the protofibrillar form of the Aβ peptide and at least one pharmaceutically acceptable carrier.

The terms "effective amount" and "therapeutically effective amount" when used in reference to a pharmaceutical composition comprising an antibody specific for the protofibrillar form of the Aβ peptide refer to an amount or dosage sufficient to produce a desired therapeutic result. More specifically, a therapeutically effective amount is an amount of antibody sufficient to prevent, at least partially, for some period of time, one or more of the clinically defined pathological processes associated with the condition being treated. The effective amount may vary depending on the specific antibody that is being used, and also depends on a variety of factors and conditions related to the subject being treated and the severity of the disorder. For example, if the antibody is to be administered *in vivo,* factors such as the age, weight, and health of the patient as well as dose response curves and toxicity data obtained in preclinical animal work would be among those factors considered. The determination of an effective amount or therapeutically effective amount of a given pharmaceutical composition is well within the ability of those skilled in the art.

The pharmaceutical composition may be appropriate for administration by the parenteral, intranasal, intravenous, intramuscular, subcutaneous, intra-cerebral and/or intraocular route.

### Antibody specific for the protofibrillar form of the Aβ peptide for use in the prevention of Alzheimer's disease

The present invention particularly relates to an antibody specific for the protofibrillar form of the Aβ peptide as defined above, for use in the prevention of Alzheimer's disease, and/or in the treatment of an early stage of symptomatic Alzheimer's disease, in particular in a subject in need thereof.

The present invention also relates to a method for preventing Alzheimer's disease and/or treating an early stage of symptomatic Alzheimer's disease in a subject in need thereof, wherein said method comprises administering to said subject an effective amount of an antibody specific for the protofibrillar form of the Aβ peptide as defined above.

The expressions "prevention of Alzheimer's disease", "treatment of an early stage of symptomatic Alzheimer's disease" and the "antibody specific for the protofibrillar form of the Aβ peptide" are particularly as defined above.

Said antibody may be provided in the form of a pharmaceutical composition, as defined above.

The subject in need thereof is as defined above in the section *"Subject to be treated".*

The subject in need for the prevention of Alzheimer's disease is preferably a subject at risk to develop asymptomatic Alzheimer's disease, for example a pre-Alzheimer subject or a subject with asymptomatic Alzheimer's disease.

The subject in need for the treatment of an early stage of symptomatic Alzheimer's disease preferably has symptomatic predementia.

The antibody is preferably used or administered by the parenteral, intranasal, intravenous, intramuscular, subcutaneous, intra-cerebral and/or intraocular route.

The doses of antibody depend on the desired effect, the duration of the treatment and the route of administration used. Generally the doctor will determine the appropriate dosage in relation to the stage of the disease, the subject's age and weight, or any other subject-related factor that has to be taken into account.

The antibody is preferably used or administered in a quantity comprised from 5 mg to 4500 mg of antibody per administration, more preferably from 100 mg to 1200 mg of antibody per administration, still more preferably from 100 mg to 450 mg of antibody per administration, particularly for an adult.

The present invention will be further illustrated in view of the following examples and figures.

All references cited herein, including journal articles or abstracts, published or unpublished patent application, issued patents or any other references, are entirely incorporated by reference herein, including all data, tables, figures and text presented in the cited references.

### Brief description of the figures

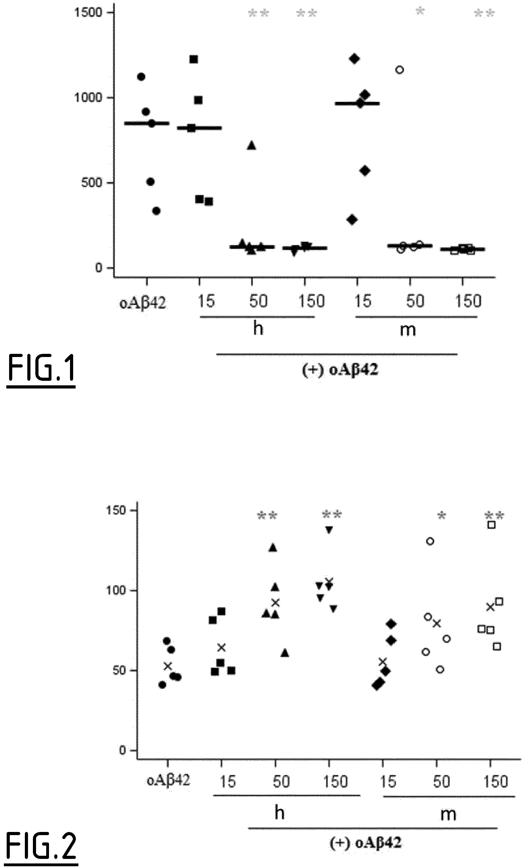

Figure 1: Inhibition of oAβ42-induced increase in caspase-3/7 enzymatic activity by humanized antibody Ab1 (h) and its murine counterpart (m) in mouse primary neuronal cultures. Ordinate: caspase 3/7 enzymatic activity (% of control). Abscissa: concentration in µg/ml. Points represent the mean by experiment and by treatment of data expressed as percentage of control group. Bars correspond to median values.* p<0.05; ** p <0.01: two-tailed Dunnett's test versus oAβ42.
Figure 2: Inhibition of oAβ42-induced decrease in neurite network by humanized antibody Ab1 (h) and its murine antibody counterpart (m) in mouse primary neuronal cultures. Ordinate: neurite network (% of control). Abscissa: concentration in µg/ml. Points represent the mean by experiment and by treatment of data expressed as percentage of control group. x correspond to mean values. *p<0.01; ** p <0.001: two-tailed Dunnett's test versus oAβ42.

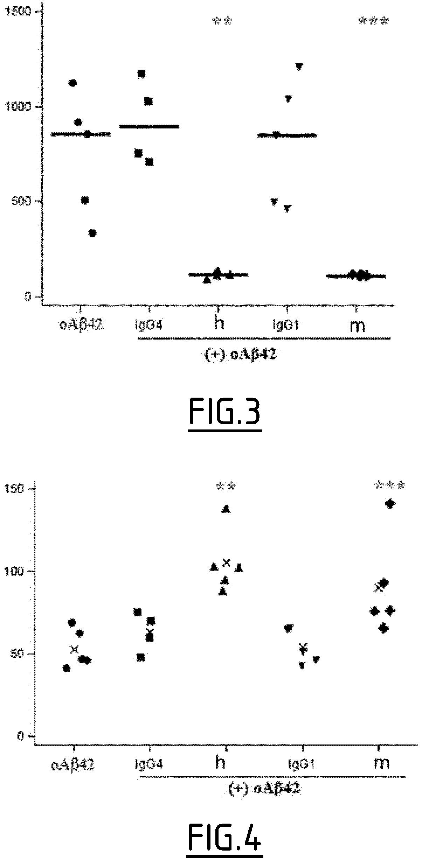

Figure 3: Comparison of the effect of humanized antibody Ab1 (h) (150 µg/mL) and its murine counterpart (m) (150 µg/mL) to that of corresponding isotype control hlgG4 (150 µg/mL) and mlgG1 (150 µg/mL) respectively on caspase-3/7 enzymatic activity in oAβ42-treated mouse primary neuronal cultures. ** p <0.001 versus IgG4. *** p <0.001 versus IgG1. p: correspond to the p-values of a contrast analysis with Bonferroni-Holm adjustment for multiplicity after a one-way analysis on rank transformed data in order to compare 7 groups to IgG corresponding groups: Ab1 and its murine counterpart at each concentration to hlgG4 and mlgG1 respectively at the same concentration in absence or presence of oAβ42. Only the results for the highest tested concentration in presence of oAβ42 are represented (Ab1 at 150µg/mL + oAβ42 versus IgG4 and its murine antibody at 150µg/mL + oAβ42 versus IgG1). Points represent the mean by experiment and by treatment of data expressed as percentage of control group. Bars correspond to median values.
Figure 4: Comparison of the effect of humanized antibody Ab1 (h) (150 µg/mL) and its murine counterpart (m) (150 µg/mL) to that corresponding isotype control hlgG4 (150 µg/mL) and mIgG1(150 µg/mL) respectively on neurite network in oAβ42-treated mouse primary neuronal cultures. ** p <0.001 versus IgG4 *** p <0.001 versus IgG1. p: correspond to the p-values of a contrast analysis with Bonferroni-Holm adjustment for multiplicity after a one-way analysis on rank transformed data in order to compare 7 groups to IgG: Ab1 and its murine counterpart at each concentration to hIgG4 and mlgG1 respectively at the same concentration in absence or presence of oAβ42. Only the results for the highest tested concentration in presence of oAβ42 are represented (Ab1 at 150µg/mL + oAβ42 versus IgG4 at 150 µg/mL and murine antibody at 150µg/mL + oAβ42 versus IgG1 at 150 µg/mL). Points represent the mean by experiment and by treatment of data expressed as percentage of control group. x correspond to mean values.

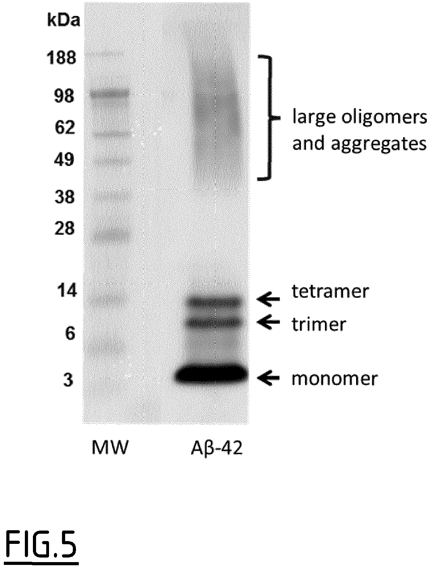

Figure 5: Western Blot of aggregated Aβ-42 (oAb42; 5 µM) preparation using anti-Aβ 4G8 mAb. MW: Molecular Weight Marker.

### Brief description of the sequences

The table 2 below summarizes the sequences of the humanized antibodies Ab1, Ab2 and Ab3 specific for the protofibrillar form of the Aβ peptide.

The number (1 or 2) following "VH", "VL", "CH" or "CL" refers herein to a sequence variant, and not for example to the conventional designation of the three constant regions CH1, CH2 and CH3.

**Table 2**

| | **Nucleotide sequences** | **Protein sequences** |
|---|---|---|
| **Antibody Ab1 VH1VL1** | | |
| VH₁ + CH₁ | SEQ ID NO: 1 | SEQ ID NO: 2 |
| VL₁+ CL₁ | SEQ ID NO: 3 | SEQ ID NO: 4 |
| VH₁ | SEQ ID NO: 5 | SEQ ID NO: 6 |
| VL₁ | SEQ ID NO: 7 | SEQ ID NO: 8 |
| CDR 1, 2, 3 of VH₁ | SEQ ID NO: 9, 11, 13 | SEQ ID NO: 10, 12, 14 |
| CDR 1, 2, 3 of VL₁ | SEQ ID NO: 15,17,19 | SEQ ID NO: 16, 18, 20 |

| **Antibody Ab2 VH1 VL2** | | |
|---|---|---|
| VH₁ + CH₁ | SEQ ID NO: 1 | SEQ ID NO: 2 |
| VL₂ + CL₂ | SEQ ID NO: 21 | SEQ ID NO: 22 |
| VH₁ | SEQ ID NO: 5 | SEQ ID NO: 6 |
| VL₂ | SEQ ID NO: 23 | SEQ ID NO: 24 |
| CDR 1, 2, 3 of VH, | SEQ ID NO: 9, 11, 13 | SEQ ID NO: 10, 12, 14 |
| CDR 1, 2, 3 of VL2 | SEQ ID NO: 31, 17, 19 | SEQ ID NO: 32, 18, 20 |

| **Antibody Ab3 VH2 VL2** | | |
|---|---|---|
| H₂ + CH₂ | SEQ ID NO: 25 | SEQ ID NO: 26 |
| VL₂ + CL₂ | SEQ ID NO: 21 | SEQ ID NO: 22 |
| VH₂ | SEQ ID NO: 27 | SEQ ID NO: 28 |
| VL₂ | SEQ ID NO: 23 | SEQ ID NO: 24 |
| CDR 1, 2, 3 of VH₂ | SEQ ID NO: 9, 11, 29 | SEQ ID NO: 10, 12, 30 |
| CDR 1, 2, 3 of VL₂ | SEQ ID NO: 31, 17, 19 | SEQ ID NO: 32, 18, 20 |

### EXAMPLES

### EXAMPLE 1: Neuronal cytoprotective effect of an antibody specific for the protofibrillar form of the Aβ peptide in vitro

### Material and methods

### (i) Animals

Cerebral cortex from 16-day old mouse embryos (OF1, Charles River Laboratories, France) were used for the preparation of primary neuronal cultures.

### (ii) Compounds

Test compounds: humanized antibody Ab1 (50 mg/mL) and its murine counterpart (30 mg/mL) were synthesized by the Global Biotherapeutics Department of Sanofi R&D, Vitry-sur-Seine, France.

Control isotypes: mouse IgG1 (antiDM4_hyb_CAA162_IgG1) (7.28 mg/mL) and human IgG4 (antiDM4_hyb_CAA162_IgG4) (4.97 mg/mL) were synthesized by the Global Biotherapeutics Department of Sanofi R&D, Vitry-sur-Seine, France.

On the day of experiment, the concentrated working solution of all antibodies and control isotypes was prepared as a 10x solution, by diluting each stock solution to 1.5 mg/mL in Dulbecco's Modified Eagle Medium (DMEM). Lower tested concentrations were prepared by diluting the concentrated working solution in DMEM. Final concentrations of tested antibody within the culture wells were 150, 50 or 15 µg/mL, ie, corresponding to 1, 0.33 or 0.1 µM respectively.

Beta Amyloid (1-42): human (Aβ42), reference 641-15 (California Peptide Research Inc, USA), was prepared as a stock solution at 1 mM in hexafluoroisopropanol, Sigma H8508 (HFIP). The resulting solution was aliquoted in microcentrifuge tubes (VWR 20170-293), then the HFIP was allowed to evaporate in the Sorbonne hood, and the resulting clear peptide film was dried under vacuum in a SpeedVac and stored dessicated at -20°C under vacuum.

On the day of experiment, the concentrated working solution was prepared from frozen HFIP-treated aliquots, resuspended at 5 mM in Dimethyl Sulfoxide (DMSO) (Sigma D2650, batch RNBB9702) by pipette mixing then followed by incubation in a ultrasonic bath for 10 minutes (Branson). Aβ42 oligomers (oAβ42) were obtained by diluting the 5 mM Aβ42 working solution to 100 µM in ice-cold cell culture medium (phenol red-free Ham's F12, Pan Biotech P04-14559), vortexing 30 seconds, and incubating 1 hour at room temperature. This type of preparation has been reported to contain Aβ42 aggregates containing mainly high-molecular weight Aβ42 oligomers.This preparation of Aβ42 was added to the culture medium at a final concentration of 5 µM.

### (iii) Primary neuronal cultures

Primary neuronal cultures were prepared from brain of 16 days-old mouse (OF1) embryos by dissecting and then dissociating in 0.25% trypsin cerebral cortices. After centrifugation at 120 g at room temperature, the resulting cell pellet resuspended in 10 mL of culture medium at a cell density of 4x105 cells/mL was plated in poly-D-lysine-coated wells of 96-well culture microplate (Greiner, 655946, Germany) in 100 µL of culture DMEM supplemented with N2 and B27.

After 6 days *in vitro,* the culture medium was removed and replaced by new one without B27, and the neurons were pretreated with anti-Aβ antibodies for 1 hour. Then the neurons were incubated with oAβ42 (5 µM) for 48 hours. Control application of drug-free medium but supplemented with the corresponding DMSO concentration (ie, 0.1%, same as for oAβ42 treatment) was run in parallel to application of test substance.

The effect of tested compounds was evaluated using the two following readouts: caspase-3/7 enzymatic activity and neurite network.

### (iv) Quantification of caspase-3/7 enzymatic activity

Following experimental treatment, 50 µL of medium was eliminated from the wells, and 50 µL of a caspase-Glo 3/7 Assay kit solution (Promega Corporation USA, G7790) were mixed to each well and then incubated for 4 hours at room temperature. After incubation the fluorescence of each sample was quantified using a Spectramax Gemini plate reader (XS, s/n XSO 2810, Molecular Devices, SoftMax PRO3.1 software) using 499 nm as the excitation wavelength and 521 nm as the emission wavelength. The measurement of fluorescence intensity is proportional to caspase-3/7 enzymatic activity.

### (v) Quantification of neurite network

### Fixation of cells

Following experimental treatment, 100 µL of a 4% formaldehyde solution (Merck, 100496) was added to each well and then incubated for 10 minutes at room temperature. The medium was then removed and replaced by 100 µL of a solution of 4% formaldehyde solution and allowed to incubate for further 10 minutes at room temperature. The medium was then removed and the wells were washed four times with 100 µL of Dulbecco's Phosphate Buffered saline (DPBS) (Gibco, 14190). The microplates were stored at 4°C in DPBS, before staining.

### Immunocytochemistry

Immunocytofluorescence procedure was applied on fixed cells in wells of culture microplates. Briefly, cell membranes were permeabilized for 30 minutes in DPBS/10% fetal bovine serum (FBS)-0.2% Triton X-100 at room temperature. Permeabilized cells were incubated overnight at 4°C with a primary antibody recognizing the neuronal protein marker MAP2 (microtubule-associated protein 2), here anti-MAP2 rabbit polyclonal antibody (Millipore AB5622, batch 2049208, 1/1 000 dilution) and then, after a washing step in PBS+0.1% Tween followed by an incubation with a secondary antibody corresponding to Alexa Fluor 594 coupled-anti-rabbit antibody (Molecular probes, A11012, batch 47098A, 1/1 000 dilution) for 1 hour at room temperature. After an additional washing step, fixed cells were then incubated 15 minutes with Hoechst 33342 (Molecular probes H3570) to stain for nuclei (0.2 µg/mL) and finally rinsed with DPBS before analysis by imaging. The measurement of neuronal associated extensions, ie, neurites was based on the MAP-2 positive immunofluorescence positive signal.

### Quantitative image analysis

Analysis of neurite network was performed on MAP-2 immunostained and Hoechst stained cells from the 96-well culture microplates using high content image analysis. Automated fluorescent image acquisition of 5 fields per well at two color excitation wavelengths was performed on the In Cell Analyzer 2200 imaging system (GE Healthcare, Cardiff, UK) equipped with a 20x high numerical aperture magnification and a 16 bit sCMOS camera in a solid-state light excitation.

The quantitative image analysis process was performed on a computer-based workstation using IN Cell Developer Toolbox software (GE Healthcare). After segmentation of nuclei (based on Hoechst staining) and neurites (based on detected MAP2 immuno-reactivity) the neurite area was measured after defining an "object mask" on each cell followed by a postprocessing step on each field acquired and automatically edited by the In Cell Developer Toolbox software in Microsoft Excel spreadsheets. The measurement of neurite area (expressed as µm2) was used as neurite network index.

The data used for the analysis are generally from five independent experiments.

### (vi) Statistical analysis

For each parameter, the data were expressed as the percentage of the control group. Then, the mean by experiment for each treatment group was calculated for the statistical analyses. Statistical analyses were performed using SAS 9.2 software. A probability less than 5% (p<0.05) was considered as significant.

### Results

The aggregated Aβ42 preparation used in the experiments for inducing neurotoxicity comprises several SDS-stable species including small and large oligomers (*see Figure 1*).

In mouse primary neuronal cultures, it was shown that oAβ42 induces a toxic effect through a marked increase in caspase 3/7 enzymatic activity (+ 648.6 %) (p=0.0105).

A significant inhibition of oAβ42-induced toxicity is observed after co-treatment with humanized antibody Ab1 at 50 and 150 µg/mL corresponding to a protective effect of 97% and 98% (p=0.0062 and p < 0.0001 versus oAβ42, respectively) (*see Figure 1*)*.*

A significant inhibition of oAβ42-induced toxicity is observed after co-treatment with the murine antibody at 50 and 150 µg/mL corresponding to a protective effect of 96% and 98% (p=0.0352 and p < 0.0001 versus oAβ42, respectively) (*see Figure 1*).

In the absence of oAβ42, no significant differences is observed between control and humanized antibody Ab1 at 50 and 150 µg/mL (p=0.5309 and p=0.4207 respectively). No significant differences is observed between control and murine antibody when tested alone at concentration of 15 and 50 µg/mL (p=0.9279 and p=0.1811 respectively) (Table 3). A statistical significant increase in caspase-3/7 enzymatic activity is observed with the murine antibody at the highest concentration of 150 µg/mL versus control (+ 15%; p=0.0352).

It was also shown that oAβ42 induces a toxic effect in mouse primary neuronal cultures through a marked decrease in neurite network in mouse primary neuronal cultures (-47 % versus control; p=0.0009).

A significant inhibition of oAβ42-induced decrease in neurite network is observed after co-treatment with oAβ42 and humanized antibody Ab1 at 50 and 150 µg/mL corresponding to a neuroprotective effect of 84% and 111 % respectively (p < 0.0001 for both concentrations versus oAβ42) (*see Figure 2*).

An apparent inhibition of oAβ42-induced decrease in neurite network is observed after co-treatment with oAβ42 and the murine antibody at 50 and 150 µg/mL (p=0.0023 and p<0.0001 respectively, versus oAβ42) (*see Figure 2*).

In the absence of oAβ42, no treatment effect in the level of neurite network is observed (p=0.3580). In the presence of oAβ42, significant differences are observed between Ab1 and isotype control hlgG4 tested at 150 µg/mL for both readouts analyzed, ie, caspase 3/7 enzymatic activity and neurite network (p=0.0007 and p<0.0001 respectively) (*see Figures 3 and 4*).

Significant differences are also observed between the murine antibody and isotype control mlgG1 at 150 µg/mL in the presence of oAβ42 for both readouts analyzed, ie, caspase 3/7 enzymatic activity and neurite network (p<0.0001 for both parameters) and at 50 µg/mL in the presence of oAβ42 for neurite network (p=0.0026) (*See Figures 3 and 4*)*.*

### Conclusion

In the assay conditions, the oAβ42 preparation is neurotoxic for mouse primary neuronal cultures, as observed by a significant and marked increase in caspase 3/7 enzymatic activity and, in parallel, by a significant decrease in neurite network.

In the assay conditions, co-treatments of oAβ42 with humanized antibody Ab1 or its murine counterpart significantly inhibit the neurotoxic effects induced by oAβ42 on mouse primary neuronal cultures, as demonstrated by the concentration-dependent inhibition of oAβ42-induced increase in caspase-3/7 enzymatic activity and of oAβ42-induced decrease in neurite network.

An antibody directed against the protofibrillar form of the Aβ peptide, such as Ab1, has surprisingly a neuro-protective activity against oAβ42-induced neurotoxicity *in vitro* and is thus useful as therapeutics for amyloid-induced pathology at the earliest stages of the disease and for the prevention of the disease.

### EXAMPLE 2: Neuronal cytoprotective effect of antibody Ab1 versus other antibodies

### Material and methods

Primary cultures of cortical neurons were dissected out from 16-day-old mouse embryoes (OF1) and plated in wells of poly-D-lysine coated 96-well culture plates (Greiner) in a Dulbecco's Modified Eagle Medium (DMEM) supplemented with N2 and B27 (40,000 cells/well/100µl).

After 6 days *in vitro,* all the medium was removed and replaced by new one without B27, and the neurons were pre-incubated with anti-Aβ antibodies for 1 hour. The neurons were then incubated with oAβ42 preparation (obtained as in Example 1), during 48 hours. Control application of drug-free medium but supplemented with the corresponding DMSO concentration (same as oAβ42) were run in parallel to applications of test substance.

The test compounds were Ab1, Ab4 (consisting of two heavy chains of sequence SEQ ID NO: 33 and two light chains of sequence SEQ ID NO: 34) and Ab5 (consisting of two heavy chains of sequence SEQ ID NO: 35 and two light chains of sequence SEQ ID NO: 36). The control antibodies were hlgG4 and hlgG1.

The concentrations of antibody were 50µg/mL or 150µg/mL.

Caspase 3/7 activity and neuritic network inhibition were assessed as in Example 1.

### Results

As regard to the two other test antibodies, Ab4 specifically binds to a conformational epitope on Aβ fibrils and Ab5 to Aβ small and large Abeta assemblies.

It was first checked that, for both parameters, Caspase 3/7 and Neuritic network, a significant difference was observed between the control without oAβ42 and the oAβ42 group and that no significant treatment effect of the test antibodies was observed in the absence of oAβ42.

As previously shown in Example 1, significant differences were observed for antibody Ab1 + oAβ42 at the tested concentrations compared to oAβ42, for both caspase 3/7 and neuritic network parameters. However, significant differences compared to oAβ42 were observed only for the neuritic network as regard to Ab4 at concentrations of 50 and 150 µg/mL and Ab5 at concentration 50 and 150 µg/mL.

## Claims

1. An antibody specific for the protofibrillar form of the Aβ peptide for use in the prevention of Alzheimer's disease and/or in the treatment of an early stage of symptomatic Alzheimer's disease.

2. The antibody for use according to claim 1, in the prevention of Alzheimer's disease in a subject who does not suffer from memory loss and/or from impaired cognitive function.

3. The antibody for use according to claim 1 or 2, in the prevention of Alzheimer's disease in a subject who does not have senile plaques, does not have neurodegeneration and/or does not have synapse dysfunction.

4. The antibody for use according to any one of claims 1 to 3, wherein said antibody prevents, at least partially, Aβ-induced cytotoxicity in neuronal cells.

5. The antibody for use according to claim 4, wherein said antibody prevents, at least partially, Aβ-induced apoptosis of neuronal cells and/or prevents, at least partially, Aβ-induced neurite network decrease.

6. The antibody for use according to any one of claims 1 to 5, wherein said antibody is a humanized antibody.

7. The antibody for use according to any one of claims 1 to 6, wherein said antibody comprises (i) the CDRs of sequence SEQ ID NO: 10, 12, 14, 16, 18 and 20, (ii) the CDRs of sequence SEQ ID NO: 10, 12, 14, 32, 18 and 20 or (iii) the CDRs of sequence SEQ ID NO: 10, 12, 30, 32, 18 and 20.

8. The antibody for use according to any one of claims 1 to 7, wherein said antibody comprises the CDRs encoded by (i) the nucleotide sequences SEQ ID NO: 9, 11, 13, 15, 17 and 19, (ii) the nucleotide sequences SEQ ID NO: 9, 11, 13, 31, 17 and 19, (iii) the nucleotide sequences SEQ ID NO: 9, 11, 29, 31, 17 and 19 or (iv) by nucleotide sequences differing by respectively 1, 2, 3, 4 or 5 nucleotides from these sequences of (i), (ii) or (iii).

9. The antibody for use according to any one of claims 1 to 8, wherein (i) the variable part of its heavy chain is encoded by a sequence having at least 80% identity with sequence SEQ ID NO: 5 or SEQ ID NO: 27 and/or (ii) the variable part of its light chain is encoded by a sequence having at least 80% identity with sequence SEQ ID NO: 7 or SEQ ID NO: 23.

10. The antibody for use according to any one of claims 1 to 9, wherein (i) the variable part of its heavy chain comprises a sequence having at least 80% identity with sequence SEQ ID NO: 6 or SEQ ID NO: 28 and/or (ii) the variable part of its light chain comprises a sequence having at least 80% identity with sequence SEQ ID NO: 8 or SEQ ID NO: 24.

11. The antibody for use according to any one of claims 1 to 10, wherein said antibody comprises (i) a heavy chain encoded by a sequence having at least 80% identity with the nucleotide sequence SEQ ID NO: 1 or SEQ ID NO: 25 and/or (ii) a light chain encoded by a sequence having at least 80% identity with the nucleotide sequence SEQ ID NO: 3 or SEQ ID NO: 21.

12. The antibody for use according to any one of claims 1 to 11, wherein said antibody comprises (i) a heavy chain having at least 80% identity with the polypeptide sequence SEQ ID NO: 2 or SEQ ID NO: 26 and/or (ii) a light chain having at least 80% identity with the polypeptide sequence SEQ ID NO: 4 or SEQ ID NO: 22.

13. The antibody for use according to any one of claims 1 to 12, wherein said antibody comprises sequences encoded by the nucleotide sequences (i) SEQ ID NO: 5 and 7, (ii) the nucleotide sequences SEQ ID NO: 5 and 23 or (iii) the nucleotide sequences SEQ ID NO: 27 and 23.

14. The antibody for use according to any one of claims 1 to 13, wherein its sequence comprises (i) the polypeptide sequences SEQ ID NO: 6 and 8, (ii) the polypeptide sequences SEQ ID NO: 6 and 24 or (iii) the polypeptide sequences SEQ ID NO: 28 and 24.

15. The antibody for use according to any one of claims 1 to 14, wherein said antibody comprises the sequences encoded by (i) the nucleotide sequences SEQ ID NO: 1 and 3, (ii) the nucleotide sequences SEQ ID NO: 1 and 21 or (iii) the nucleotide sequences SEQ ID NO: 25 and 21.

16. The antibody for use according to any one of claims 1 to 15, wherein said antibody comprises (i) the polypeptide sequences SEQ ID NO: 2 and 4, (ii) the polypeptide sequences SEQ ID NO: 2 and 22 or (iii) the polypeptide sequences SEQ ID NO: 26 and 22.
